(19) Europäisches Patentamt
European Patent Office
Office européen des brevets

(11) Veröffentlichungsnummer: **0 135 770**
**A2**

(12) # EUROPÄISCHE PATENTANMELDUNG

(21) Anmeldenummer: **84109639.9**

(22) Anmeldetag: **13.08.84**

(51) Int. Cl.[4]: **C 07 D 487/04**, A 61 K 31/55
// (C07D487/04, 243:00, 235:00)

(30) Priorität: **25.08.83 US 526194**

(43) Veröffentlichungstag der Anmeldung: **03.04.85**
**Patentblatt 85/14**

(84) Benannte Vertragsstaaten: **AT BE CH DE FR GB IT LI LU NL SE**

(71) Anmelder: **F. HOFFMANN-LA ROCHE & CO. Aktiengesellschaft, CH-4002 Basel (CH)**

(72) Erfinder: **Fryer, Rodney Ian, 5 Eton Drive, North Caldwell, N.J. (US)**
Erfinder: **Walser, Armin, 19 Crane Avenue, West Caldwell, N.J. (US)**

(74) Vertreter: **Lederer, Franz, Dr. et al, Patentanwälte Dr. Franz Lederer Dipl.-Ing. Reiner F. Meyer-Roxlau Lucile-Grahn-Strasse 22, D-8000 München 80 (DE)**

(54) **Imidazobenzodiazepinderivate, Verfahren zu deren Herstellung und Arzneimittel enthaltend diese Verbindungen.**

(57) Die Imidazobenzodiazepine der Formel

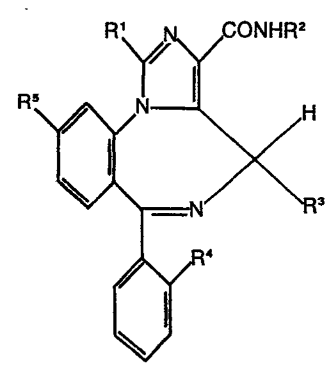

I

worin $R^1$ Wasserstoff, Di-nieder Alkylamino-nieder Alkyl oder nieder Alkyl, $R^2$ Wasserstoff nieder Alkanoyl oder nieder Alkyl, $R^3$ Wasserstoff, nieder Alkanoyloxy oder Hydroxy, $R^4$ Chlor, Brom, Bluor oder Trifluormethyl und $R^5$ Wasserstoff, Fluor oder Chlor bedeuten, mit den Maßgaben, daß a) $R^3$ nicht Hydroxy bedeuten kann, falls $R^2$ nieder Alkanoyl bedeutet und b) $R^3$ Wasserstoff bedeutet, fall $R^1$ Di-nieder Alkylamino-nieder Alkyl bedeutet,

und die pharmazeutisch verwendbaren Salze davon besitzen eine anxiolytische Wirkung, ohne daß sie unerwünschte CNS-Nebenwirkungen besitzen, wie übermäßige Sedation, Äthanolpotenzierung und Ataxie, und können demnach als selektive Anxiolytika verwendet werden. Sie sind bereits generisch offenbart, beispielsweise in der U.S. Patentschrift 4 280 957, und können nach verschiedenen Methoden hergestellt werden.

EP 0 135 770 A2

0135770

F.HOFFMANN-LA ROCHE & CO.Aktiengesellschaft, Basel/Schweiz

13. Aug. 1984

RAN 4008/332

# Imidazobenzodiazepinderivate, Verfahren zu deren Herstellung und Arzneimittel enthaltend diese Verbindungen

Die vorliegende Erfindung betrifft Imidazobenzodiazepinderivate der allgemeinen Formel

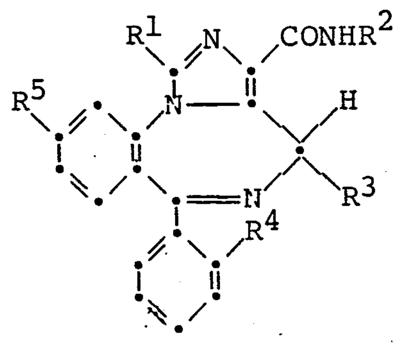

I

worin $R^1$ Wasserstoff, Di-nieder Alkylamino-nieder Alkyl oder nieder Alkyl, $R^2$ Wasserstoff nieder Alkanoyl oder nieder Alkyl, $R^3$ Wasserstoff, nieder Alkanoyloxy oder Hydroxy, $R^4$ Chlor, Brom, Fluor oder Trifluormethyl und $R^5$ Wasserstoff, Fluor oder Chlor bedeuten, mit den Massgaben, dass a) $R^3$ nicht Hydroxy bedeuten kann, falls $R^2$ nieder Alkanoyl bedeutet und b) $R^3$ Wasserstoff bedeutet, falls $R^1$ Di-nieder Alkylamino-nieder Alkyl bedeutet,

und die pharmazeutisch verwendbaren Säureadditionssalze davon.

Die Verbindungen der obigen Formel I besitzen eine anxiolytische Wirkung, ohne dass sie die nachteiligen Nebenwirkungen CNS-aktiver Verbindungen aufweisen, wie übermässige Sedation und Ataxie.

Die Verbindungen der Formel I sind in der am 28. Juli 1981 publizierten U.S. Patentschrift 4,280,957 als Teil einer breiten generischen Erfindung offenbart und beansprucht. Erst im nachhinein wurde festgestellt, dass die Verbindung der Formel I, ein Subgenus der ursprünglichen Erfindung, selektive anxiolytische Wirkung besitzen und die bei vielen der gegenwärtig kommerzialisierten Anxiolytika gefundenen unerwünschten Nebeneffekte, wie übermässige Sedation, Aethanolpotenzierung und Ataxie, nicht aufweisen.

Der in dieser Beschreibung verwendete Ausdruck "nieder Alkanoyl" umfasst den Acylrest einer geradkettigen oder verzweigten gesättigten aliphatischen Carbonsäure mit 1-4 Kohlenstoffatomen, wie z.B. Formyl, Acetyl, Propionyl und dgl. Der Ausdruck "nieder Alkanoyloxy" betrifft einen niederen Alkanoylrest, der eine substituierte Sauerstoffunktion aufweist, wie z.B. Acetoxy, Propionyloxy und dgl.

Der in dieser Beschreibung verwendete Ausdruck "nieder Alkyl" - allein oder in Kombination - betrifft geradkettige, verzweigte und cyclische Kohlenwasserstoffreste mit 1-7 Kohlenstoffatomen, vorzugsweise geradkettige oder verzweigte Kohlenwasserstoffreste mit 1-4 Kohlenstoffatomen, wie Methyl, Aethyl, Propyl, Isopropyl, Butyl, t-Butyl und dgl.

Der Ausdruck "pharmazeutisch verwendbare Salze" betrifft Salze mit anorganischen und organischen, pharmazeutisch verwendbaren Säuren, wie Schwefelsäure, Salzsäure,

Salpetersäure, Methansulfonsäure und p-Toluolsulfonsäure. Solche Salze können von einem Fachmann nach bekannten Methoden und in Abhängigkeit von der Art der Verbindung, die man in ein Salz überführen will, leicht hergestellt werden.

Eine bevorzugte Klasse von Verbindungen der obigen Formel I sind solche, worin $R^1$ Wasserstoff, $R^2$ Wasserstoff oder Aethyl, $R^4$ Chlor oder Fluor und $R^5$ Wasserstoff bedeuten.

Speziell bevorzugte Verbindungen der Formel I sind beispielsweise:

9-Chlor-6-(2-chlorphenyl)-4H-imidazo[1,5-a][1,4]benzodiazepin-3-carboxamid,

6-(2-Chlorphenyl)-4H-imidazo[1,5-a][1,4]benzodiazepin-3-carboxamid,

6-(2-Chlorphenyl)-1-methyl-4H-imidazo[1,5-a][1,4]benzodiazepin-3-carboxamid,

6-(2-Chlorphenyl)-4-hydroxy-4H-imidazo[1,5-a][1,4-benzodiazepin-3-carboxamid und

N-Acetyl-6-(2-chlorphenyl)-4H-imidazo[1,5-a][1,4]benzodiazepin-3-carboxamid.

Die Verbindungen der Formel I und deren pharmazeutisch verwendbaren Salze können erfindungsgemäss dadurch hergestellt werden, dass man

a) eine Verbindung der allgemeinen Formel

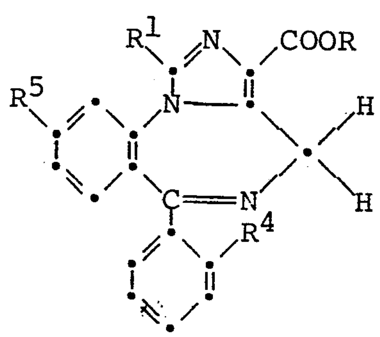

XII

worin R niederes Alkyl bedeutet und $R^1$, $R^4$ und $R^5$ obige Bedeutung besitzen,

einer Aminolyse mit einer Aminoverbindung der Formel $H_2NR^{21}$, worin $R^{21}$ Wasserstoff oder niederes Alkyl bedeutet, unterwirft, oder

b) eine Verbindung der allgemeinen Formel

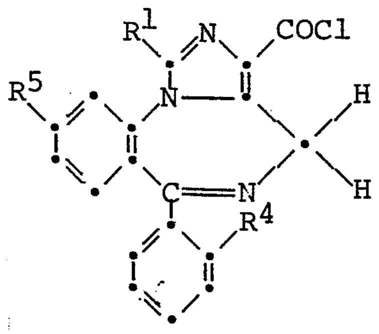

XVI

worin $R^1$, $R^4$ und $R^5$ die obige Bedeutung besitzen,

einer Aminolyse mit einer Aminoverbindung der Formel $H_2NR^{21}$, worin $R^{21}$ obige Bedeutung besitzt, unterwirft, oder

c) eine Verbindung der allgemeinen Formel

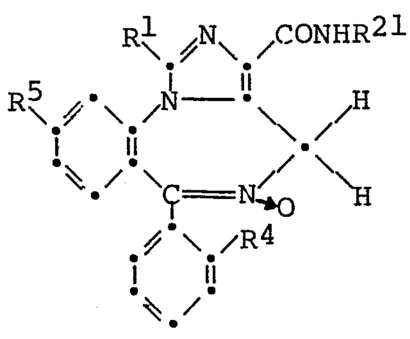

XVII

worin $R^1$, $R^{21}$, $R^4$ und $R^5$ die obige Bedeutung besitzen,

mit einem $C_1$-$C_4$-Carbonsäureanhydrid umsetzt, oder

d) eine Verbindung der allgemeinen Formel

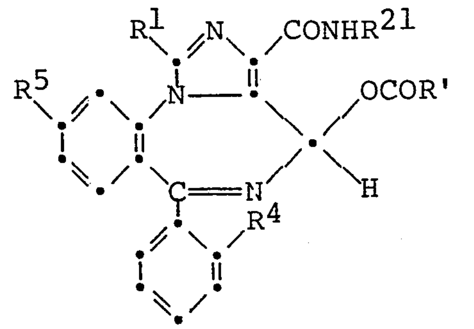

Ib'

worin R' $C_1$-$C_4$-Alkyl bedeutet und $R^1$, $R^{21}$, $R^4$ und $R^5$ die obige Bedeutung besitzen,
mit einem Alkalimetallhydroxyd umsetzt, oder

e) eine Verbindung der allgemeinen Formel

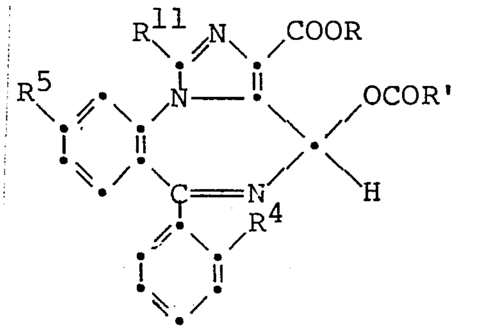

XVIII

worin $R^{11}$ Wasserstoff oder nieder Alkyl bedeutet und R, R', $R^4$ und $R^5$ obige Bedeutung besitzen,
einer Aminolyse mit einer Aminoverbindung der Formel $H_2NR^{21}$, worin $R^{21}$ die obige Bedeutung besitzt, unterwirft, oder

f) eine Verbindung der allgemeinen Formel

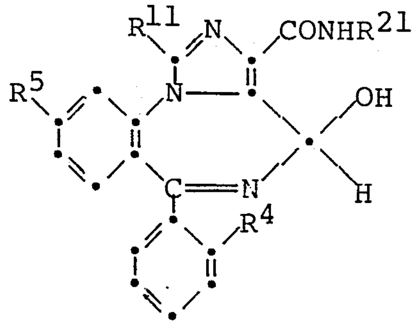

Ib''

worin $R^{11}$, $R^{21}$, $R^4$ und $R^5$ obige Bedeutung besitzen,

mit einem $C_1$-$C_4$-Carbonsäurechlorid oder -anhydrid umsetzt, oder

g) eine Verbindung der allgemeinen Formel

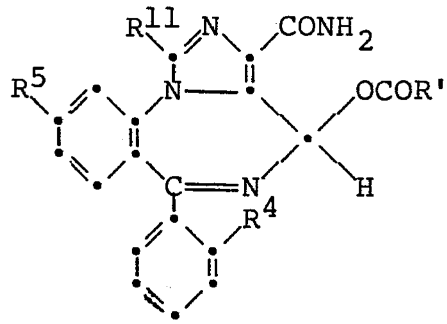

Ic'

worin R', $R^{11}$, $R^4$ und $R^5$ die obige Bedeutung besitzen,

mit einem $C_1$-$C_4$-Carbonsäurechlorid oder -anhydrid umsetzt, und

h) erwünschtenfalls eine erhaltene Verbindung der Formel I in ein pharmazeutisch verwendbares Salz überführt.

Die folgenden allgemeinen Reaktionsschemata A-D illustrieren die Reaktionen, welche zur Herstellung von Verbindungen der Formel I brauchbar sind. In diesen Reaktionsschemata bedeuten R nieder Alkyl, R' $C_1$-$C_4$-Alkyl, $R^{11}$ und $R^{21}$ Wasserstoff oder nieder Alkyl, $R^{12}$ Di-nieder Alkylaminomethyl, $R^{31}$ nieder Alkanoyloxy oder Hydroxy, X Halogen und Z Benzyloxycarbonyl und besitzen $R^1$, $R^2$, $R^4$ und $R^5$ die in Formel I angegebene Bedeutung. Die Reaktionen gemäss Schema A können auch mit den entsprechenden N-Oxyden durchgeführt werden, aber ein in den Verbindungen der Formel VI vorliegender N-Oxydrest wird während der Umwandlung VI → VII entfernt werden.

## Reaktionsschema A

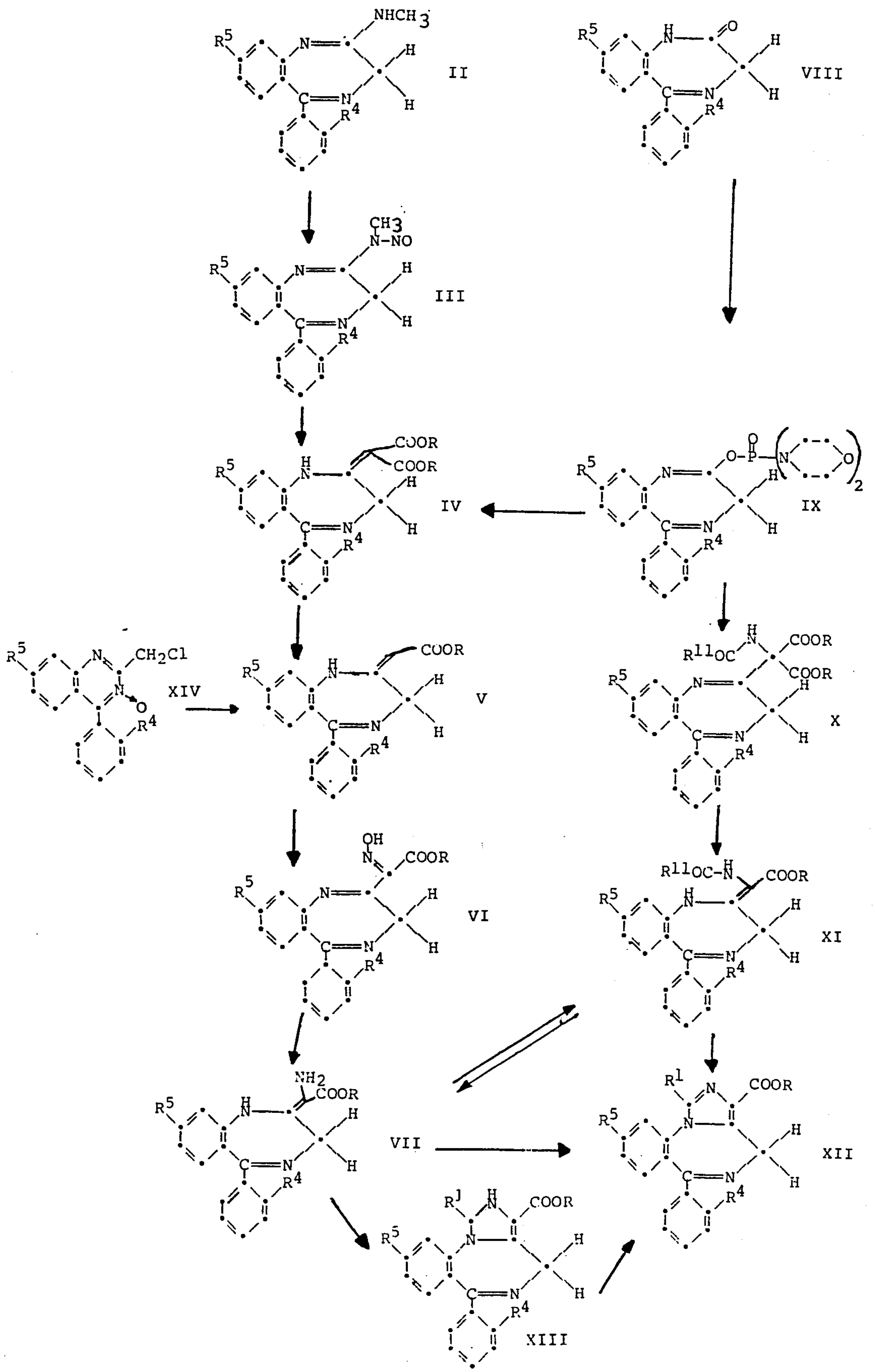

Reaktionsschema B

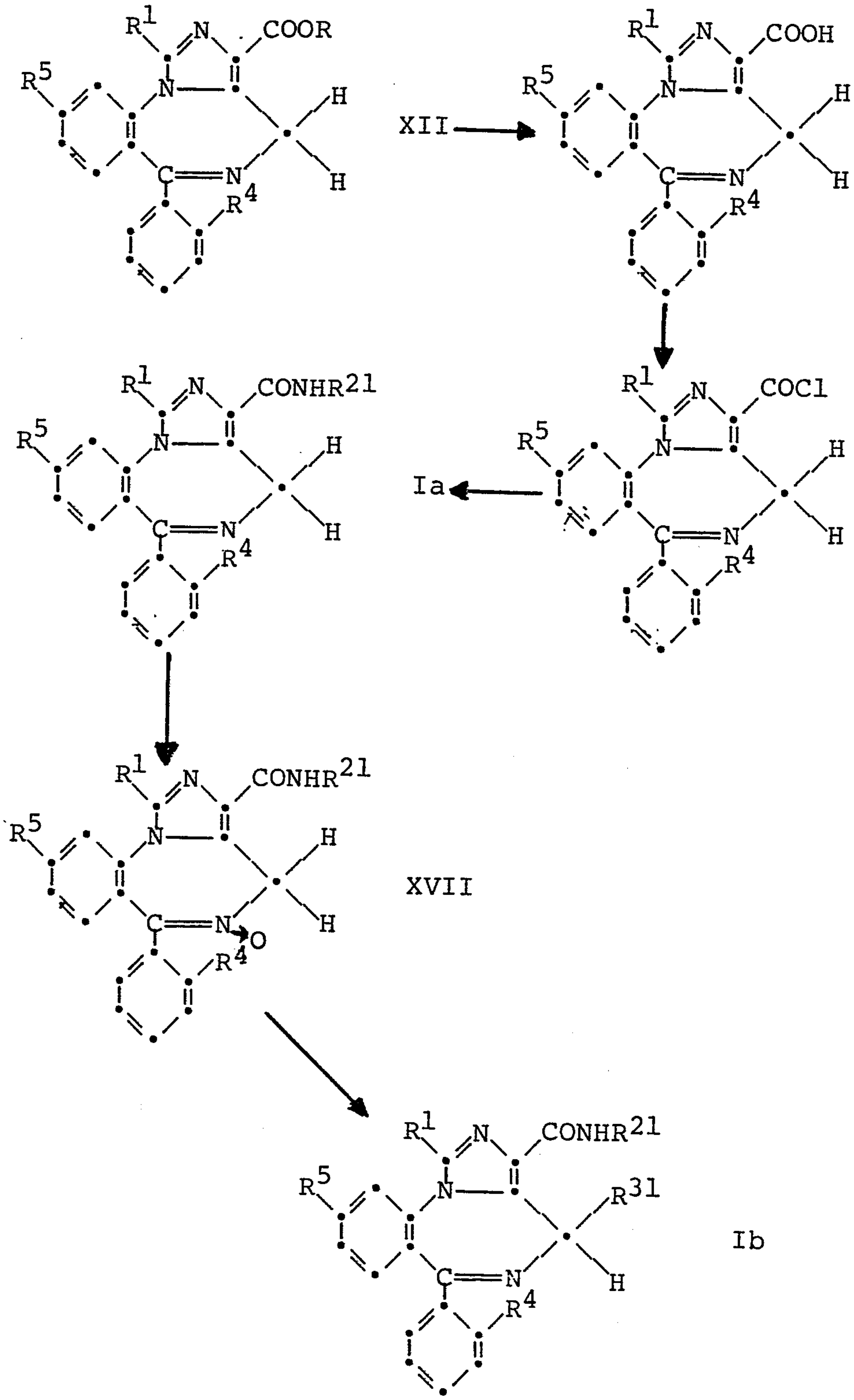

XII

XV

XVI

Ia

XVII

Ib

Reaktionsschema C

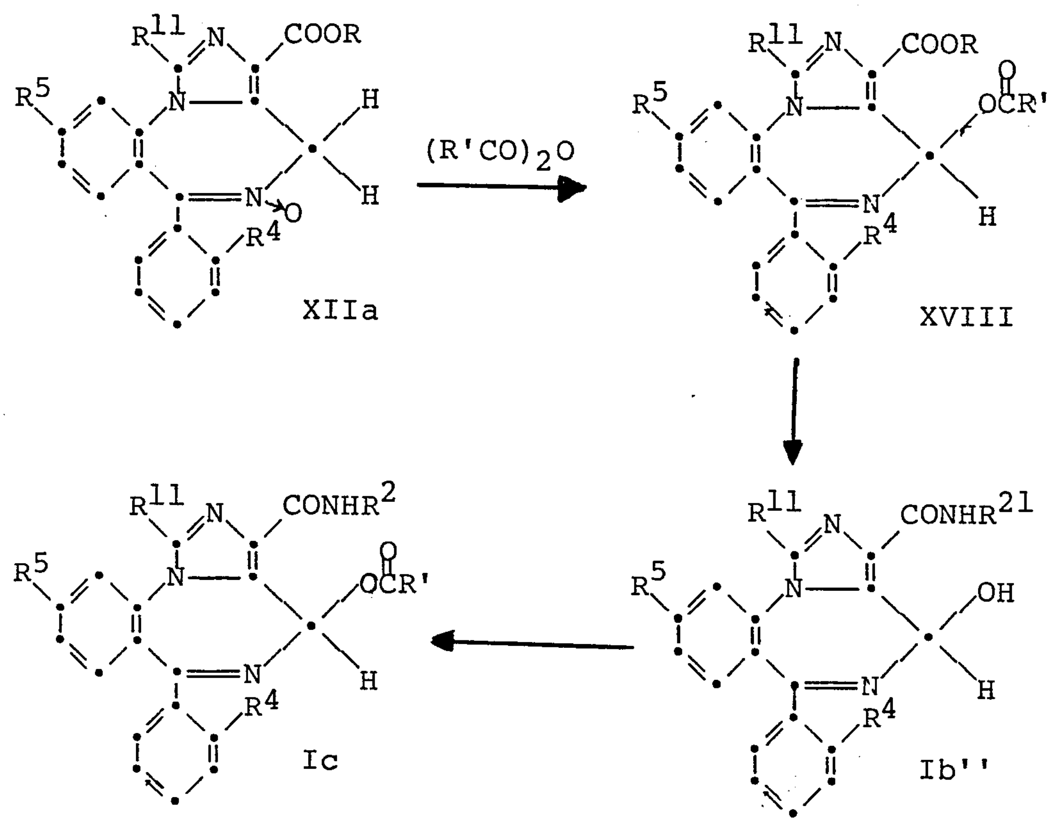


XIIa

XVIII

Ib''

Ic

**Reaktionsschema D**

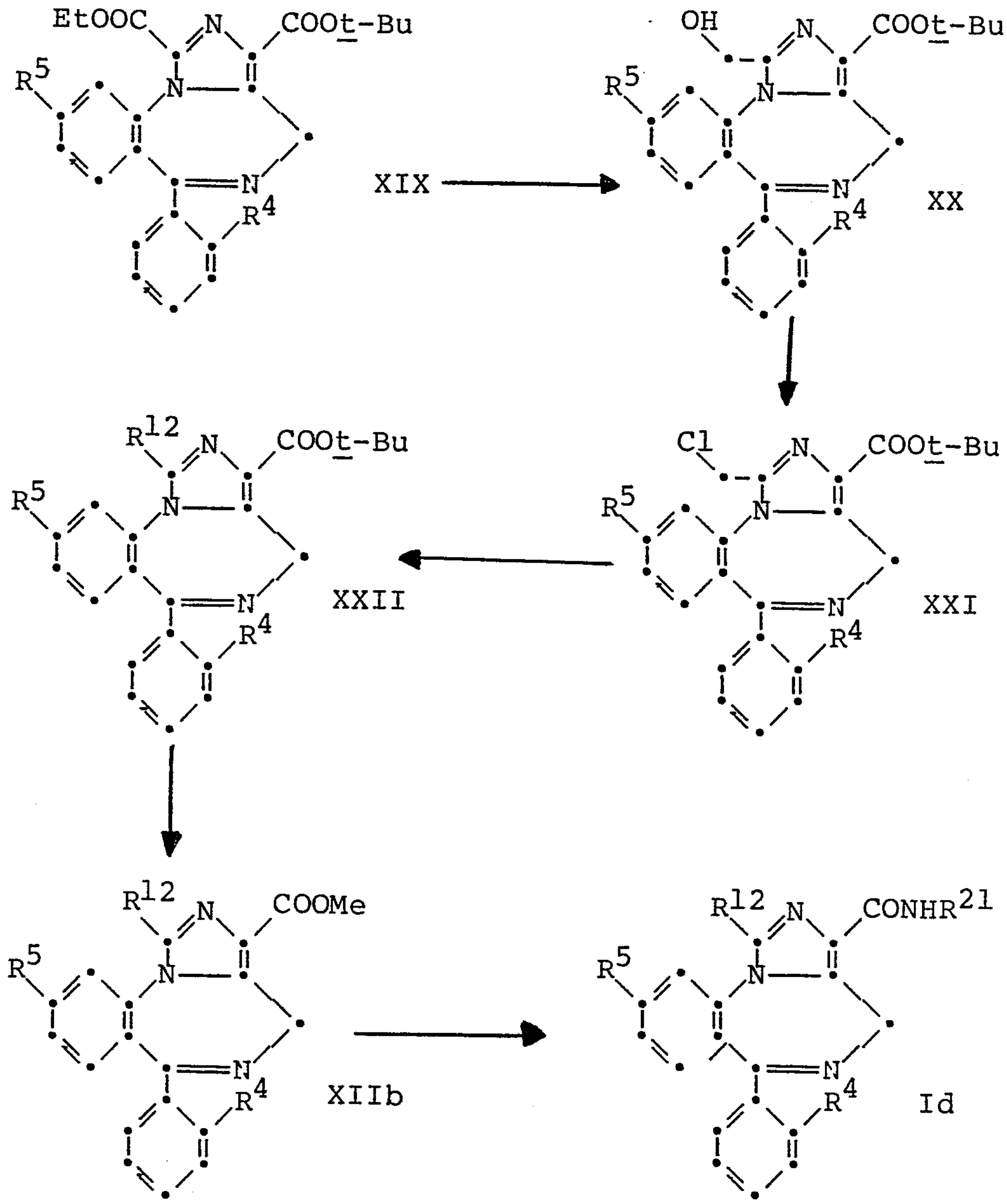

XIX

XX

XXI

XXII

XIIb

Id

Stufe II → III

Verbindungen der Formel III werden durch Nitrosierung von Verbindungen der Formel II hergestellt. Eine solche Nitrosierung kann durch in situ gebildete salpetrige Säure bewirkt werden. Reagenzien, welche verwendet werden können sind (1) Alkalimetallnitrite, beispielsweise Natriumnitrit, in Gegenwart von organischen oder anorganischen Säuren, beispielsweise Eisessig, und wässrigen oder nicht wässrigen Lösungsmitteln; (2) Alkylnitrite, beispielsweise Methylnitrit in Gegenwart eines inerten Lösungsmittels, wie eines Alkohols, eines chlorierten Kohlenwasserstoffes oder beispielsweise Dimethylformamid; und (3) eine Nitrosylchloridgas-Lösung in einem inerten Lösungsmittel in Gegenwart eines Säureazzeptors wie Pyridin. Eine solche Nitrosierungsreaktion sollte bei oder unterhalb Raumtemperatur, d.h. im Bereich von -20° bis 25°C durchgeführt werden.

Stufe VIII → IX

Verbindungen der Formel IX können durch Reaktion der Verbindungen der Formel VIII mit Dimorpholinophosphinchlorid hergestellt werden. Die für die Herstellung von Verbindungen der Formel IX notwendig Umsetzung von Verbindungen der Formel VIII mit dem Phosphorylierungsmittel wird so durchgeführt, dass man eine Verbindung der Formel VIII mit einer Base umsetzt, welche stark genug ist, die Verbindung der Formel VIII unter Bildung des entsprechenden Anions zu ionisieren. Geeignete Basen umfassen Alkalimetallalkoxyde, wie Kalium-t-butoxyd oder Natriummethoxyd, Alkalimetallhydride, wie Natriumhydrid, und Alkyllithiumverbindungen, wie n-Butyllithium. Die Reaktionstemperatur liegt im Bereich von 0° bis 100°C, und die Reaktion wird vorzugsweise in einem aprotisch polaren inerten Lösungsmittel durcheführt, d.h. einem Lösungsmittel, welches die umgebenen Salze der Verbindung der Formel VIII vollkommen oder zumindest teilweise solubilisiert. Bevorzugte Lösungsmittel sind Aether, z.B. Tetrahydrofuran oder Dioxan, oder tertiäre Amide, z.B.

Dimethylformamid.

Stufe III oder IX → IV

Verbindungen der Formel III oder IX können mit dem Anion, das sich von einem Malonsäureester der Formel

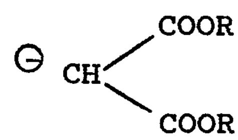

ableitet, unter Bildung einer Verbindung der Formel IV kondensiert werden. Das Anion wird durch Deprotonierung des Malonsäureesters mit einer geeigneten starken Base, wie Alkalimetall- oder Erdalkalimetallalkoxyden, Hydriden oder Amiden gebildet. Die Reaktion der Verbindung der Formel III oder IX mit dem Malonsäureesteranion wird vorzugsweise in einem Lösungsmittel, wie einem Kohlenwasserstoff, z.B. Benzol, Toluol oder Hexan, einem Aether, z.B. Dioxan, Tetrahydrofuran oder Diäthyläther, Dimethylformamid, Dimethylsulfoxyd usw. bei einer Temperatur im Bereich von unterhalb Raumtemperatur bis 150°C, vorzugsweise 0° bis 100°C, am meisten bevorzugt bei Raumtemperatur durchgeführt.

Stufe IV → V

Verbindungen der Formel V werden durch Decarboxylierung von Verbindungen der Formel IV erhalten, indem man eine Verbindung der Formel IV mit einem Alkalimetallhydroxyd, wie Natrium- oder Kaliumhydroxyd in einem geeigneten Lösungsmittel, wie einem Alkohol, Aether oder Dimethylsulfoxyd bei einer Temperatur im Bereich zwischen etwa Raumtemperatur und Rückflusstemperatur, vorzugsweise 60° bis 100°C, umsetzt.

Stufe XIV → V

Gemäss einem Alternativverfahren kann eine Verbindung der Formel V auch hergestellt werden, indem man eine Verbindung der Formel XIV mit einem Essigsäureester, d.h.

einer Verbindung der Formel $CH_3COOR$, in Gegenwart einer Base umsetzt, die genügend stark ist, um das Proton vom Ester zu entfernen, wobei das Anion des ausgewählten Essigsäureesters gebildet wird. Ein geeignetes Beispiel einer solchen Base ist Lithiumdiisopropylamid.

Stufe V → VI

Verbindungen der Formel VI werden durch Nitrosierung von Verbindungen der Formel V hergestellt, indem diese mit salpetriger Säure umsetzt, die z.B. aus einem Alkalimetallnitrit, Alkylnitrit oder Nitrosylchlorid durch Umsetzen mit einer organischen oder anorganischen Säure hergestellt wird. Geeignete Lösungsmittel für die Nitrosierungsreaktion sind Aether, Alkohole, Wasser, Säuren, z.B. Essigsäure, Dimethylformamid, Dimethylsulfoxyd und chlorierte Kohlenwasserstoffe. Die Reaktion kann bei Raumtemperatur durchgeführt werden, obwohl die Temperatur nicht kritisch ist.

Stufe VI → VII

Verbindungen der Formel VII werden durch Reduktion von Verbindungen der Formel VI mit z.B. Raney-nickel und Wasserstoff oder Zink und Essigsäure hergestellt. Diese Reduktion liefert hauptsächlich Verbindungen der Formel VII und gleichzeitig kleine Mengen verschiedener möglicher Isomerer, d.h. Verbindungen der Formeln

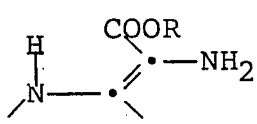

VII A

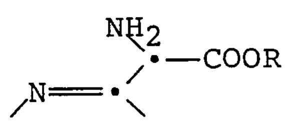

VII B

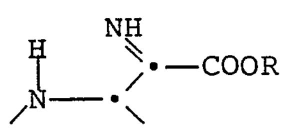

VII C

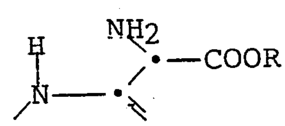

VII D

Stufe VII → XII

Verbindungen der Formel XII entstehen dann durch Reaktion einer Verbindung der Formel VII mit einem Alkansäureorthoester der Formel

$$R^{11}C(OR)_3$$

gegebenenfalls in Gegenwart eines Säurekatalysators, z.B. einer organischen oder anorganischen Säure, z.B. p-Toluolsulfonsäure, Phosphorsäure usw. bei Raumtempratur oder darüber, d.h. 25-150°C, wobei unter diesen Bedingungen die Cyclisation zur Verbindung XII spontan eintritt. Technische Aequivalente des oben genannten Orthoesters sind Orthoamide, z.B. Dimethylacetal von N,N-Dimethylformamid, N,N,N',N',N",N"-Hexamethylenmethantriamin, Nitrile, z.B. Acetonitril, Esterimidate, z.B. $CH_3$-C(=NH)-$OC_2H_5$.

Stufe VII → XI

Verbindungen der Formel XI können Acylierung von Verbindungen der Formel VII mit einer Verbindung der Formel

$$R^{11}COX \quad \text{oder} \quad (R^{11}CO)_2O$$

hergestellt werden. Lösungsmittel für diese Reaktionsstufe sind Methylenchlorid, Aether, chlorierte Kohlenwasserstoffe usw., vorzugsweise in Kombination mit einem Säureakzeptor, wie einer organischen oder anorganischen Base, wie Triäthylamin, Pyridin oder einem Alkalimetallcarbonat. Die Reaktion kann oberhalb oder unterhalb der Raumtemperatur durchgeführt werden, vorzugsweise jedoch bei Raumtemperatur. Verbindungen der Formel XI sind isomer, d.h. sie können die folgenden stereochemischen Strukturen aufweisen:

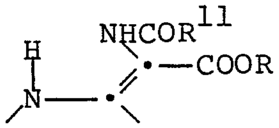

XI A

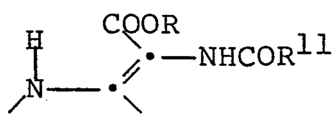

XI B

Stufe XI → XII

Verbindungen der Formel XII können auch durch Dehydratisierung von Verbindungen der Formel XI oder Isomeren davon unter gleichzeitiger Cyclisation durch Erhitzen hergestellt werden. Dieser Reaktionsschritt kann mit oder ohne Lösungsmittel, z.B. Dimethylformamid, Aethylenglykol, Hexamethylphosphorsäuretriamid, bei einer Temperatur im Bereich von 100° bis 300°C, vorzugsweise 150° bis 250°C, beispielsweise 200°C mit oder ohne Katalysator und Wasserbindemitteln durchgeführt werden.

Stufe IX → X

Verbindungen der Formel X können hergestellt werden, indem man eine Verbindung der Formel IX mit dem Anion, das sich von einem Acylaminomalonsäureester der Formel

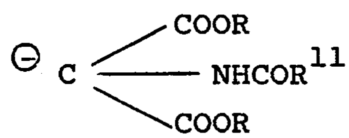

ableitet, umsetzt. Das Anion wird durch Deprotonierung des Acylaminomalonsäureesters mit einer geeigneten starken Base, wie Alkalimetall- oder Erdalkalimetallalkoxyden, -hydriden oder -amiden erzeugt. Die Umsetzung einer Verbindung der Formel IX mit dem Acylaminomalonsäureesteranion wird vorzugsweise in einem Lösungsmittel, wie einem Kohlenwasserstoff, z.B. Benzol, Toluol oder Hexan, einem Aether, z.B. Dioxan, Tetrahydrofuran oder Diäthyläther, Dimethylformamid, Dimethylsulfoxyd usw. bei einer Temperatur im Bereich von unterhalb Raumtemperatur bis 150°C, vorzugsweise 0° bis 100°C, am bevorzugtesten bei Raumtemperatur, durchgeführt.

Stufe X → XI

Verbindungen der Formel XI und Isomere davon werden durch Decarboxylierung von Verbindungen der Formel X mit einem Alkalimetallalkoxyd in einem Lösungsmittel, wie einem

Aether, Alkohol, Dimethylsulfoxyd, Dimethylformamid usw. oberhalb oder unterhalb von Raumtemperatur, vorzugsweise bei Raumtemperatur, hergestellt. Die Verbindungen der Formeln X und XI brauchen nicht isoliert zu werden, sondern können in situ in die Verbindungen der Formel XII übergeführt werden.

Stufe VII → XIII

Verbindungen der Formel XIII werden durch Umsetzen einer Verbindung der Formel VII mit einem Aldehyd der Formel $R^1CHO$ erhalten. Für diese Reaktionsstufe geeignete Lösungsmittel sind Kohlenwasserstoffe, wie Benzol, Alkohole, Aether, chlorierte Kohlenwasserstoffe, Dimethylformamid, Dimethylsulfoxyd usw. Die Umsetzung kann mit oder ohne Wasserbindemittel, z.B. Molekularsieb, oberhalb oder unterhalb von Raumtemperatur, vorzugsweise zwischen Raumtemperatur und Rückflusstemperatur des Lösungsmittels durchgeführt werden.

Eine weitere Methode zur Herstellung von Verbindungen der Formel XIII, worin $R^1$ Di-nieder Alkylaminoäthyl bedeutet, besteht in der Kondensation einer Verbindung der Formel VII mit einem Di-nieder Alkylamin, z.B. Dimethylamin, zusammen mit Acrolein.

Stufe XIII → XII

Verbindungen der Formel XIII können durch Oxidation in situ mit Oxidationsmitteln, wie Mangandioxyd, Luft, Sauerstoff usw., in Verbindungen der Formel XII übergeführt werden.

Ein anderes Verfahren zur Herstellung von Verbindungen der Formel XII besteht zunächst in der Umsetzung einer Verbindung der Formel IX mit einem geschützten Aminomalonsäureester der Formel

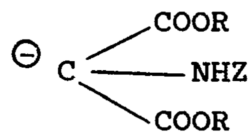

Ueberführung der so erhaltenen Verbindung, welche der Formel X entspricht, worin $R^{11}$ jedoch Benzyloxy bedeutet, in eine Verbindung, welche der Formel XI entspricht, wie oben für die Stufe X → XI angegeben und schliesslich Behandlung der so erhaltenen Verbindung mit Bromwasserstoff in Eisessig unter Bildung einer Verbindung der Formel VII. Die Zwischenprodukte der Formeln X und XI brauchen nicht isoliert zu werden. Die so erhaltene Verbindung der Formel VII wird weiter in die Verbindung der Formel XII übergeführt, und zwar über die oben beschriebenen Reaktionsstufen VII → XIII und XIII → XII.

Nach noch einem anderen Verfahren können die Verbindungen der Formel XII, worin $R^1$ Wasserstoff bedeutet, hergestellt werden, indem man eine Verbindung der Formel III oder IX oder eine Verbindung, die diesen Formeln III und IX entspricht, welche jedoch in 2-Stellung eine andere geeignete Abgangsgruppe trägt, wie eine Di-nieder Alkoxyphosphonogruppe -OPO(OAlkyl)$_2$, mit einem Isocyanoacetat der Formel C N-$CH_2$-COOR in Gegenwart einer Base umsetzt, welche basisch genug ist, das Anion des Isocyanoacetats zu bilden. Die Umsetzung erfolgt in einem inerten Lösungsmittel, wie Dimethylformamid, Hexamethylphosphorsäuretriamid, Dimethylsulfoxyd, Tetrahydrofuran oder jedem anderen geeigneten organischen Lösungsmittel. Alles was vom organischen Lösungsmittel im obigen Schritt verlangt wird, ist, dass die Ausgangsstoffe darin löslich sind und dass das Lösungsmittel nicht in die Reaktion eingreift. Die Reaktionstemperatur liegt zwischen etwa -40°C und etwa Raumtemperatur, vorzugsweise zwischen etwa 0°C und Raumtemperatur.

Basen, welche stark genug sind, das Anion des Isocyanoacetats zu bilden, sind Alkalimetallalkoxyde, wie Kalium-t-butoxyd oder Natriummethoxyd, Alkalimetallhydride, wie Natriumhydrid, und Alkalimetallamide, wie Lithiumamid oder Lithiumdiisopropylamid.

Stufe XII → XV

Verbindungen der Formel XV werden durch Hydrolyse von Verbindungen der Formel XII zu den entsprechenden Säuren, vorzugsweise mit Alkalimetallhydroxyden, z.B. Natrium- oder Kaliumhydroxyd, hergestellt. Die Hydrolyse wird zweckmässigerweise in einem inerten Lösungsmittel durchgeführt. Geeignete Lösungsmittel sind Alkohole, z.B. Methanol oder Aethanol, Aether, z.B. Dioxan oder Tetrahydrofuran, Dimethylformamid, in Kombination mit Wasser. Vorzugsweise wird diese Reaktionsstufe bei einer Temperatur zwischen Raumtemperatur und dem Siedepunkt der Reaktionsmischung durchgeführt.

Stufe XII oder XV → Ia

Verbindungen der Formel Ia können durch direkte Aminolyse von Verbindungen der Formel XII (mit einer Aminoverbindung der Formel $H_2NR^{21}$) oder durch Umwandeln einer Verbindung der Formel XV in das Säurechlorid XVI, beispielsweise durch Behandeln mit Phosphorpentachlorid, und nachfolgendem Umsetzen mit einer Aminoverbindung der Formel $H_2NR^{21}$, hergestellt werden.

Die Stufe XII → Ia wird zweckmässigerweise in einem inerten Lösungsmittel oder in Abwesenheit eines Lösungsmittels durchgeführt. Geeignete Lösungsmittel sind Kohlenwasserstoffe, z.B. Hexan oder Toluol, Aether, z.B. Tetrahydrofuran, Alkohole, z.B. Methanol oder Aethanol, Dimethylformamid, Dimethylsulfoxyd, Hexamethylphosphorsäuretriamid. Es ist bevorzugt, diese Reaktionsstufe bei einer Temperatur zwischen etwa 50° und 200°C, besonders bevorzugt zwischen etwa 100° und 150°C, bei Atmosphärendruck oder einem Druck

oberhalb Atmosphärendruck durchzuführen.

Die Stufe XV → Ia via XVI wird zweckmässigerweise in einem inerten Lösungsmittel durchgeführt. Geeignete Lösungsmittel sind Kohlenwasserstoffe, z.B. Hexan oder Toluol, Aether, z.B. Tetrahydrofuran, chlorierte Kohlenwasserstoffe, z.B. Methylenchlorid oder Chlorbenzol. Vorzugsweise wird diese Reaktion bei einer Temperatur zwischen etwa -20°C und dem Siedepunkt der Reaktionsmischung, besonders bevorzugt zwischen etwa 0° und 50°C, durchgeführt.

Stufe Ia → XVII

Verbindungen der Formel XVII werden durch Umwandlung der entsprechenden Verbindungen Ia in die N-Oxyde davon hergestellt. Die Umwandlung wird durch Umsetzen einer Verbindung der Formel Ia mit einer organischen Persäure bewirkt. Eine übliche organische Persäure, wie Peressigsäure, Perpropionsäure, m-Chlorperbenzoesäure usw. kann bei der Durchführung dieser Reaktionsstufe verwendet werden. Die Oxidation kann bei Raumtemperatur oder oberhalb oder unterhalb Raumtemperatur erfolgen.

Stufe XVII → Ib

Verbindungen der Formel XVII können dann zur Herstellung von Verbindungen der Formel Ib nach bekannten Methoden verwendet werden, wie z.B. eine Polonovski-Umlagerung unter Verwendung eines Säureanhydrids unter Bildung des nieder Alkanoyloxyrestes, welcher dann durch Behandeln mit einem Alkalimetallhydroxyd, wie Natriumhydroxyd in die Hydroxygruppe übergeführt werden kann. Ein Beispiel einer solchen Polonovski-Umlagerung ist in der U.S. Patentschrift 3,296,249 beschrieben.

Stufe XIIa → XVIII

Verbindungen der Formel XVIII werden erhalten, indem man ein N-Oxyd der Formel XIIa mit einem $C_1$-$C_4$-Carbonsäureanhydrid, vorzugsweise Essigsäureanhydrid, in Gegenwart oder Abwesenheit eines inerten organischen Lösungsmittels, wie Xylol oder Toluol, bei einer Reaktionstemperatur zwischen etwa 100°C und dem Siedepunkt des verwendeten Lösungsmittelsystems umsetzt, wobei die Reaktionstemperatur vorzugsweise beim Siedepunkt des verwendeten Lösungsmittels durchgeführt wird. Es ist darauf hinzuweisen, dass diese Umsetzung erwünschtenfalls auch ohne Lösungsmittel durchgeführt werden kann, d.h. mit dem Anhydrid allein.

<u>Stufe XVIII → Ib"</u>

Verbindungen der Formel Ib" können hergestellt werden, indem man eine Verbindung der Formel XVIII mit Ammoniak oder einem nieder Alkylamin, z.B. einem $C_1$-$C_4$-Alkylamin, vorzugsweise Methyl- oder Aethylamin, in einem $C_1$-$C_4$-Alkohol, vorzugsweise Methanol, oder erwünschtenfalls in der Aminkomponente selbst umsetzt. Die Reaktionstemperatur liegt zwischen etwa 0° und 100°C, wobei etwa Raumtemperatur bevorzugt ist.

<u>Stufe Ib" → Ic</u>

Die Verbindungen der Formel Ib" werden mit einem nieder Alkansäurechlorid oder -anhydrid mit 1-4 Kohlenstoffatomen in Gegenwart eines Säureazzeptors, wie Pyridin, umgesetzt. Lässt man die erhaltene Verbindung der Formel Ic, worin $R^2$ Wasserstoff bedeutet, noch weiter mit dem nieder Alkansäurechlorid oder -anhydrid, beispielsweise Essigsäurechlorid oder -anhydrid, reagieren, so kann das als $R^2$ bezeichnete Wasserstoffatom durch eine nieder Alkanoylgruppe ersetzt werden. Die Reaktion wird vorzugsweise unter Rückflussbedingungen durchgeführt.

<u>Stufe XIX → XX</u>

Eine Verbindung der Formel XIX kann unter Verwendung analoger Herstellungsverfahren wie sie für die Herstellung von Verbindungen der Formel XII beschrieben sind, hergestellt werden, ausgehend von der früher beschriebenen Verbindung der Formel V. Der Diester der Formel XIX kann mit

Natriumborhydrid in einem siedenden Alkanol, z.B. Aethanol, zur Verbindung der Formel XX reduziert werden.

Stufe XX → XI

Die Verbindung der Formel XX kann danach mit Thionylchlorid umgesetzt werden, wobei die Hydroxygruppe durch ein Chloratom ersetzt wird.

Stufe XXI → XXII

Die Verbindung der Formel XXI kann dann mit einem Di-nieder Alkylamin, z.B. Dimethylamin, bei etwa 100°C umgesetzt werden, wobei man eine Verbindung der Formel XXII erhält.

Stufe XXII → XIIb

Der t-Butylestersubstituent einer Verbindung der Formel XXII kann in den entsprechenden Methylester übergeführt werden durch Umsetzen mit methanolischer Schwefelsäure.

Stufe XIIb → Id

Die Ueberführung einer Verbindung der Formel XIIb kann durchgeführt werden, wie sie oben für die Stufe XII Ia beschrieben ist, z.B. durch Umsetzen dieser Verbindung mit methanolischem Ammoniak in Gegenwart von Ammoniumchlorid oder einem Alkylamin in Gegenwart von dessen Hydrochloridsalz.

Die Verbindungen der Formel I sind anxiolytisch wirksam. Diese Aktivität kann mit den folgenden Tests gezeigt werden, wobei die bevorzugten Verbindungen der vorliegenden Erfindung eingesetzt werden. Es ist darauf hinzuweisen, dass die erfindungsgemässen Verbindungen im Rotierstabtest, welcher für eine Aussage bezüglich muskelrelaxierender Wirkung relevant ist, inaktiv oder praktisch inaktiv sind, während sie im Antimetrazoltest, welcher für eine Aussage bezüglich anxiolytischer Wirkung relevant ist, eine hohe

Aktivität besitzen.

Rotierstabtest

Dieser Test ist eine Modifikation der von N.W. Dunham und T.S. Miya in J.A. Ph. A. Sci. Ed. 46, 208 (1957) beschriebenen Methode. CF-1-Mäuse, welche aufgrund ihrer Fähigkeit ausgewählt worden sind, sich während 2 Minuten auf einem Rotierstab von 30 mm Durchmesser und 16 Umdrehungen pro Minute halten zu können, werden verwendet. Gruppen von je 8 Mäusen werden 30 Minuten nach Verabreichung der Testverbindung auf den Rotierstab verbracht und während 2 Minuten beobachtet. Diejenigen Mäuse, die sich nicht während der vollen 2 Minuten auf dem Rotierstab halten können, werden als durch die Testverbindung beeinflusst qualifiziert. Die Anzahl derjenigen Mäuse, die sich auf dem Rotierstab nicht halten können, wird in Prozent der Gesamtanzahl Mäuse in jeder Gruppe bei den verschiedenen Dosen angegeben. Diese Prozentwerte werden gegen die Dosis auf logarithmischem Papier aufgezeichnet, und der $ED_{50}$-Wert wird nach der Methode von L.C. Miller und M.L. Tainter in Proc. Soc. Exp. Biol. Med. 57, 26 (1944) ermittelt.

Der Zweck des vorliegenden Tests ist die Ueberprüfung von chemischen Verbindungen im Hinblick auf ihre Wirkung auf den Muskeltonus und/oder die muskuläre Koordination, speziell diejenige von Muskelrelaxantien, Sedativa oder Stimulantien.

| Aktivität und Standardverbindungen: | $ED_{50}$ (mg/kg p.o.) |
|---|---|
| Chlorpromazin | 10,0 ± 0,5 |
| Methotrimeprazin | 3,8 ± 0,4 |
| Chlordiazepoxyd | 33,5 ± 8,0 |
| Diazepam | 7,4 ± 0,9 |

Resultate:

| Verbindungen | $ED_{50}$ (mg/kg p.o.) |
|---|---|
| 9-Chlor-6-(2-chlorphenyl)-4H-imidazo-[1,5-a][1,4]benzodiazepin-3-carboxamid | >200 |
| 6-(2-Chlorphenyl)-1-methyl-4H-imidazo-[1,5-a][1,4]benzodiazepin-3-carboxamid | >200 |
| 6-(2-Chlorphenyl)-4-hydroxy-4H-imidazo-[1,5-a][1,4]benzodiazepin-3-carboxamid | >200 |
| N-Acetyl-6-(2-chlorphenyl)-4H-imidazo-[1,5-a][1,4]benzodiazepin-3-carboxamid-acetylester | >200 |
| 6-(2-Chlorphenyl)-4H-imidazo[1,5-a][1,4]benzodiazepin-3-carboxamid | >200 |

Intravenöser Antimetrazoltest

Männliche CF-1-Mäuse, welche 45-54 Tage alt sind und während ungefähr 24 Stunden vom Futter ferngehalten wurden, werden in diesem Test eingesetzt. Die Testverbindung, dispergiert in 5% Akazienöl, wird 3 Mäusen oral verabreicht und zwar bei einer Dosis, die 1/10 der im TSP-Test ermittelten Lethaldosis entspricht. Eine Stunde später wird Metrazol intravenös verabreicht (70 mg/kg; konvulsive Dosis 100 mg/kg) und die Versuchstiere während 30 Sekunden beobachtet, ob ein Schutz gegenüber den Krampfanfällen eintritt. Falls die Testverbindung oral aktiv ist, werden pro Dosis 3 Versuchstiere eingesetzt. Die Dosis, bei der 50% der Versuchstiere keinerlei Krampfanfälle erleiden, wird als $ED_{50}$-Wert bezeichnet.

Gemacht Messungen: Die Anzahl der Versuchstiere, welche gegen Krampfanfälle geschützt sind.

Berechnungen: Falls die Prüfsubstanz aktiv ist und 3 Mäuse pro Dosis eingesetzt werden, wird der approximative $ED_{50}$-Wert nach der Methode von Miller und Tainter (Proc. Soc. Exp. Biol. med. 57:261 (1944)) berechnet. Falls 6 oder mehr Mäuse pro Dosis eingesetzt werden, wird der $ED_{50}$--

Wert sowie der 95%ige Vertrauensbereich mit Hilfe eines Computerprogramms berechnet, welches auf der Methode von D.J. Finney (Probit Analysis, Cambridge University Press, Cambridge, England, 1971) beruht.

Aktivität von Standardverbindungen

| Verbindung | $ED_{50}$ (mg/kg p.o.) |
|---|---|
| Chlordiazepoxyd | 3,9 |
| Diazepam | 1,0 |
| Natriumphenobarbital | 19 |

Dieser Test eignet sich zur Evaluation von Antikonvulsiva und ist für eine Aussage bezüglich der anxiolytischen Wirkung relevant.

Resultate:

| Verbindung | $ED_{50}$ (mg/kg p.o.) |
|---|---|
| 9-Chlor-6-(2-chlorphenyl)-4H-imidazo[1,5-a][1,4]benzodiazepin-3-carboxamid | 0,97 |
| N-Acetyl-6-(2-chlorphenyl)-4H-imidazo[1,5-a][1,4]benzodiazepin-3-carboxamid | 1,3 |
| 6-(2-Chlorphenyl)-1-methyl-4H-imidazo[1,5-a][1,4]benzodiazepin-3-carboxamid | 5,1 |
| 6-(2-Chlorphenyl)-4-hydroxy-4H-imidazo[1,5-a][1,4]benzodiazepin-3-carboxamid | 0,86 |
| 6-(2-Chlorphenyl)-4H-imidazo[1,5-a]-[1,4]benzodiazepin-3-carboxamid | 7,4 |

Die erfindungsgemässen Imidazobenzodiapine sind wertvolle Pharmazeutika und zeichnen sich durch eine selektive anxiolytische Wirkung aus. Diese Verbindungen können in

Form von herkömmlichen pharmazeutischen Dosierungsformen verabreicht werden. Beispielsweise können diese Verbindungen mit üblichen organischen oder anorganischen, für die parenterale oder enterale Verabreichung geeigneten inerten pharmazeutischen Trägermaterialien vermischt werden, wie z.B. Wasser, Gelatine, Milchzucker, Stärke, Magnesiumstearat, Talk, pflanzliche Oele, Gummi, Polyalkylenglykole, Vaseline oder dgl. Sie können in üblichen pharmazeutischen Formen verabreicht werden, z.B. in fester Form, z.B. als Tabletten, Dragées, Kapseln, Suppositorien oder dgl. oder in flüssiger Form, z.B. als Lösungen, Suspensionen oder Emulsionen. Darüberhinaus können die pharmazeutischen, erfindungsgemässe Verbindungen enthaltenden Kompositionen üblichen pharmazeutischen Operationen wie Sterilisieren unterworfen werden und sie können auch noch herkömmliche pharmazeutische Excipientien enthalten, wie Konservierungsmittel, Stabilisierungsmittel, Emulgiermittel, Salze zum variieren des osmotischen Druckes oder Puffer. Die Kompositionen können auch noch andere therapeutisch wertvolle Verbindungen enthalten.

Eine geeignete pharmazeutische Dosierungseinheit kann von ungefähr 0,1 bis ungefähr 500 mg eines Imidazobenzodiazepinendprodukts enthalten, wobei ein Dosierungsbereich von ungefähr 0,1 mg bis ungefähr 100 mg für die orale Verabreichung bevorzugt ist, während ein Dosierungsbereich von ungefähr 0,1 bis ungefähr 50 mg für die parenterale Verabreichung bevorzugt ist. Jedoch sollte für jeden einzelnen die spezifische Dosierung festgesetzt werden, in Abhängigkeit von den individuellen Bedürfnissen und dem Urteil derjenigen Person, welche die Komposition verabreicht oder die Verabreichung überwacht. Es sei jedoch an dieser Stelle betont, dass die oben genannten Dosierungen lediglich als Beispiele angegeben wurden und dass sie den Umfang der vorliegenden Erfindung in keiner Weise beschränken.

Der Ausdruck "Dosierungseinheit" wie er in dieser Beschreibung verwendet wird, bezieht sich auf einzelne pharmazeutische Einheiten, die als Einheitsdosen für Warmblütler geeignet sind und die neben dem notwendigen pharmazeutischen Verdünnungsmittel, Träger oder Trägerstoff eine genau bestimmte Menge an aktivem Wirkstoff enthalten, die nach Berechnungen notwendig ist, den erwünschten therapeutischen Effekt zu erzielen.

Die nachfolgenden Beispiele veranschaulichen die vorliegende Erfindung, beschränken diese jedoch in keiner Weise. Alle Temperaturen sind in Celsiusgraden angegeben.

## Beispiel 1

6-(2-Chlorphenyl)-4H-imidazo[1,5-a][1.4]benzodiazepin-3-carbonsäure-äthylester

9 g (0,08 Mol) Kalium-t-butoxyd werden zu einer Lösung von 19 g (0,07 Mol) 5-(2-Chlorphenyl)-1,3-dihydro-2H-1,4-benzodiazepin-2-on in 350 ml Dimethylformamid gegeben, welches auf 5° abgekühlt ist. Nach 5-minütigen Rühren unter Stickstoff werden 18,1 g Diäthylchlorphosphat zugegeben, und das Gemisch weitere 10 Minuten gerührt. Danach werden 12,7 g (0,112 Mol) Aethylisocyanoacetat in 150 ml Dimethylformamid zugegeben, gefolgt von 12,6 g Kalium-t-butoxyd. Nach 1-stündigem Rühren ohne Kühlung wird das Reaktionsgemisch mit Eisessig angesäuert und zwischen Toluol und gesättigter Natriumbicarbonatlösung verteilt. Die organische Phase wird getrocknet und eingedampft. Kristallisation aus Aether liefert 6-(2-Chlorphenyl)-4H-imidazo[1,5-a][1,4]-benzodiazepin-3-carbonsäure-äthylester, Schmelzpunkt 225-227°. Für die Analyse wird er aus Methylenchlorid/-Aethylacetat umkristallisiert, wobei man leicht gelbliche Kristalle erhält, Smp. 226-228°.

## Beispiel 2

6-(2-Fluorphenyl)-4H-imidazo[1,5-a][1,4]benzodiazepin-3-carbonsäure-äthylester

2,5 g (23 mMol) Kalium-t-butoxyd werden zu einer Lösung von 5,08 g (20 mMol) 5-(2-Fluorphenyl)-1,3-dihydro-2H-1,4-benzodiazepin-2-on in 75 ml auf 0° gekühltes Tetrahydrofuran gegeben. Nach 5-minütigem Rühren werden 4,3 ml (30 mMol) Diäthylchlorphosphat zugegeben und das Rühren unter Stickstoff 15 Minuten fortgesetzt. Eine Lösung von 3,4 ml (32 mMol) Aethylisocyanoacetat wird dann zugegeben, unmittelbar gefolgt von 3,5 g (32 mMol) Kalium-t-butoxyd. Das Gemisch wird bei Raumtemperatur 2 Stunden gerührt, mit Eisessig angesäuert und zwischen Wasser und Methylenchlorid verteilt. Die organische Phase wird getrocknet und eingedampft, und der Rückstand aus Aether kristallisiert, wobei man 6-(2-Fluorphenyl)-4H-imidazo[1,5-a][1,4]benzodiazepin-3-carbonsäure-äthylester erhält. Für die Analyse wird dieser aus Aether umkristallisiert (Smp. 197-198°).

## Beispiel 3

6-(2-Bromphenyl)-4H-imidazo[1,5-a][1,4]benzodiazepin-3-carbonsäure-äthylester

4,5 g (40 mmOl) Kalium-t-butoxyd werden zu einer Lösung von 11 g (35 mMol) 5-(2-Bromphenyl)-1,3-dihydro-2H-1,4-benzodiazepin-2-on in 300 ml in Eiswasser gekühltem Dimethylformamid gegeben. Unter Stickstoff und Rühren werden 7,6 ml (9 g, 52 mMol) Diäthylchlorphosphat zugegeben. Nach 5-minütigem Rühren werden 7,9 g (70 mMol) Aethylisocyanoacetat und 7,8 g (70 mMol) Kalium-t-butoxyd zugegeben. Das Gemisch wird bei Raumtemperatur 2 Stunden gerührt, mit Eisessig angesäuert und zwischen Toluol und Wasser verteilt. Die organische Phase wird mit Wasser gewaschen, getrocknet und eingedampft. Der Rückstand wird mit 10% (V/V) Aethylacetat in Methylenchlorid über Silicagel filtriert. Das Filtrat wird eingedampft, und der Rückstand aus Aether

kristallisiert, wobei man 6-(2-Bromphenyl)-4H-imidazo-[1,5-a][1,4]benzodiazepin-3-carbonsäure-äthylester erhält, Smp. 230-232°. Eine Analysenprobe wird aus Methylenchlorid/Aethylacetat umkristallisiert, wobei man farblose Kristalle vom gleichen Smp. erhält.

Das in Beispiel 3 als Ausgangsmaterial verwendete Benzodiazepin-2-on kann wie folgt hergestellt werden (Beispiele 4-8):

## Beispiel 4

N-[2-[(2-Bromphenyl)hydroxymethyl]phenyl]-N-(phenylmethyl)-2-chloracetamid

17,5 g (0,15 Mol) Bortrichlorid werden in 100 ml Methylenchlorid gelöst und unter Eiskühlung zu einer Lösung von 27,45 g (0,15 Mol) N-(Phenyl)benzylamin in 400 ml Methylenchlorid gegeben. Nach Zugabe von 15,1 g (0,15 Mol) Triäthylamin wird das Reaktionsgemisch 2 Stunden zum Rückfluss erhitzt. Unter Kühlen mit Eiswasser wird eine Lösung von 27,75 g (0,15 Mol) 2-Brombenzaldehyd und 19,7 g (0,195 Mol) Triäthylamin in 400 ml Methylenchlorid langsam zugegeben. Nach 18-stündigem Rühren bei Raumtemperatur wird das Reaktionsgemisch mit 10%iger wässriger Natriumcarbonatlösung gewaschen, getrocknet und eingedampft. Der ölige Rückstand wird in 1 l Methylenchlorid gelöst, und 32 g Chloracetylchlorid werden zur in Eiswasser gekühlten Lösung gegeben. Das Reaktionsgemisch wird mit 10%iger wässriger Natriumcarbonatlösung versetzt und das Zweiphasensystem 30 Minuten heftig gerührt. Die organische Phase wird getrocknet und eingedampft. Kristallisation aus Aethylacetat/Aether trennt ein Nebenprodukt ab, welches abfiltriert wird. Das Filtrat wird eingedampft, und der Rückstand aus Aether kristallisiert, wobei man N-[2-[(2-Bromphenyl)hydroxymethyl]-phenyl]-N-(phenylmethyl)-2-chloracetamid in drei Fraktionen erhält. Dieses Material wird ohne weitere Reinigung oxydiert. Eine Analysenprobe wird durch Umkristallisation aus

0135770

Methylenchlorid/Hexan erhalten und schmilzt bei 163-166°. NMR ($CDCl_3$) zeigt ein Gemisch von zwei Rotameren.

Beispiel 5

N-[2-(2-Brombenzoyl)phenyl]-N-(phenylmethyl)-2-chloracetamid

Ein Gemisch von 27,7 g (62 mMol) N-[2-[(2-Bromphenyl)-hydroxymethyl]phenyl]-N-(phenylmethyl)-2-chloracetamid, 140 g Mangandioxyd und 1,4 l Methylenchlorid wird bei Raumtemperatur 5 Stunden gerührt. Das Mangandioxyd wird über Celit abfiltriert, und das Filtrat eingedampft. Kristallisation des Rückstandes aus 2-Propanol liefert N-[2-(2-Brombenzoyl)phenyl]-N-(phenylmethyl)-2-chloracetamid, Smp. 120-123°.

Beispiel 6

2-Azido-N-[2-(2-brombenzoyl)phenyl]-N-(phenylmethyl)acetamid

Ein Gemisch von 16,7 g (37,5 mMol) N-[2-(2-Brombenzoyl)phenyl]-N-(phenylmethyl)-2-chloracetamid, 3,45 g (53 mMol) Natriumazid und 200 ml Dimethylformamid wird 45 Minuten auf 65-76° erhitzt. Dann wird es zwischen Wasser und Methylenchlorid verteilt. Die organische Phase wird getrocknet und eingedampft, und der Rückstand aus Aether/-Hexan kristallisiert, wobei man 2-Azido-N-[2-(2-brombenzoyl)phenyl]-N-(phenylmethyl)acetamid erhält, Smp. 93-95°.

Beispiel 7

5-(2-Bromphenyl)-1,3-dihydro-1-(phenylmethyl)-2H-1,4-benzodiazepin-2-on

Ein Gemisch von 14,8 g (33 mMol) 2-Azido-N-[2-(brombenzoyl)phenyl]-N-(phenylmethyl)acetamid, 200 ml Tetrahydrofuran, 350 ml Aethanol und 15 g Raney-nickel wird bei Atmosphärendruck während 1,5 Stunden hydriert. Der Katalysator wird abfiltriert, und das Filtrat 30 Minuten zum Rückfluss erhitzt, nachdem man 10 ml Eisessig zugegeben

hat. Die Lösung wird eingedampft, und der Rückstand zwischen Methylenchlorid und 10%iger wässriger Natriumcarbonatlösung verteilt. Die organische Phase wird getrocknet und eingedampft. Kristallisation des Rückstandes aus 2-Propanol liefert 5-(2-Bromphenyl)-1,3-dihydro-1-(phenylmethyl)-2H-1,4-benzodiazepin-2-on als farbloses Produkt, Smp. 132-134°. Für die Analyse wird es aus 2-Propanol umkristallisiert, Smp. 135-138°.

## Beispiel 8

5-(2-Bromphenyl)-1,3-dihydro-2H-1,4-benzodiazepin-2-on

Eine Lösung von 6,6 g (16,3 mMol) 5-(2-Bromphenyl)-1,3-dihydro-1-(phenylmethyl)-2H-1,4-benzodiazepin-2-on in 125 ml konz. Schwefelsäure wird 12 Minuten auf einem Dampfbad erhitzt. Das abgekühlte Reaktionsgemisch wird auf Eis gegossen und mit Ammoniak alkalisch gestellt. Der ausgefällte Niederschlag wird mit Methylenchlorid/Aethanol extrahiert. Die Extrakte werden getrocknet und über Silicagel filtriert, wobei man zum Auswaschen 5% (V/V) Aethanol in Methylenchlorid verwendet. Das Filtrat wird eingedampft und der Rückstand aus Methylenchlorid/Aethylacetat/Hexan kristallisiert, wobei man 5-(2-Bromphenyl)-1,3-dihydro-2H-1,4-benzodiazepin-2-on als farblose Kristalle erhält, Smp. 220-222°.

## Beispiel 9

6-[2-(Trifluormethyl)phenyl]-4H-imidazo[1,5-a][1,4]benzodiazepin-3-carbonsäure-äthylester

Umsetzen von 8 g (26,3 mMol) 1,3-Dihydro-5-(2-trifluormethyl)phenyl-2H-1,4-benzodiazepin-2-on mit Kalium-t-butoxyd und Diäthylchlorphosphat und nachfolgendes Behandeln mit dem Anion von Aethylisocyanat liefert nach Kristallisation aus Methylenchlorid/Aether 6-[2-(Trifluormethyl)phenyl]-4H-imidazo[1,5-a][1,4]benzodiazepin-3-carbonsäure-äthylester. Dieser wird aus Methylenchlorid/Aethanol zu

Analysenzwecken umkristallisiert und schmilzt bei 236-238°.

## Beispiel 10

9-Fluor-6-(2-fluorphenyl)-4H-imidazo[1,5-a][1.4]benzodiazepin-3-carbonsäure-äthylester

Eine Lösung von 0,82 g (3 mMol) 8-Fluor-5-(2-fluorphenyl)-1,3-dihydro-2H-1,4-benzodiazepin-2-on in 20 ml Tetrahydrofuran wird auf -10° gekühlt und unter Stickstoff gerührt, während man 0,4 g (3,56 mMol) Kalium-t-butoxyd zugibt. Nach 5 Minuten gibt man 0,61 g (0,51 ml oder 3,56 Mol) Diäthylchlorphosphat zu und rührt 10 Minuten weiter. Man gibt dann 0,4 ml Aethylisocyanat zu, gefolgt von 0,4 g (3,56 mMol) Kalium-t-butoxyd. Nach Entfernen der Kühlung wird das Gemisch 30 Minuten gerührt, mit Essigsäure angesäuert und zwischen Methylenchlorid und Natriumbicarbonatlösung verteilt. Die organische Phase wird getrocknet und eingedampft. Kristallisation des Rückstandes aus Aether liefert 9-Fluor-6-(2-fluorphenyl)-4H-imidazo[1,5-a][1.4]-benzodiazepin-3-carbonsäure-äthylester. Dieser wird aus Aethylacetat/Hexan zu Analysenzwecken umkristallaisiert und schmilzt bei 215-217°.

Das in Beispiel 10 als Ausgangsmaterial verwendete Benzodiazepin-2-on kann nach der Indolmethode wie folgt hergestellt werden (Beispiele 11-15):

## Beispiel 11

6-Fluor-3-(2-fluorphenyl)-1H-indol-2-carbonsäure-methylester

Eine Lösung von 30,4 g (0,44 Mol) Natriumnitrit in 200 ml Wasser wird tropfenweise bei -20 bis -5° zu einem Gemisch von 44,4 g (0,4 Mol) 3-Fluoranilin in 160 ml konz. Salzsäure gegeben. Nach beendigter Zugabe wird diese Lösung während 15 Minuten bei -5 bis 0° gerührt und danach auf ein wie folgt hergestelltes Gemisch gegossen: 90 g (0,4 Mol) 2-Acetyl-3-(2-fluorphenyl)propionsäure-methylester wird in

320 ml Methanol gelöst, und die erhaltene Lösung auf -50° gekühlt. Eine Lösung von 76 g (1,9 Mol) Natriumhydroxyd in 200 ml Wasser wird zugegeben, während man die Temperatur unterhalb -5° hält. Nach Vereinigung der beiden Reaktionsgemische unterhalb 0° wird das neue Reaktionsgemisch ohne Kühlung 45 Minuten gerührt. Danach wird es mit Wasser verdünnt und mit Methylenchlorid extrahiert. Die Extrakte werden getrocknet und eingedampft. Das rote Oel wird in 1 l Methanol gelöst, und die Lösung mit Chlorwasserstoffgas gesättigt. Nach 2-stündigem Erhitzen zum Rückfluss wird die Lösung mit Wasser verdünnt, und die ausgefallenen Kristalle abgetrennt. Umkristallisation aus Aether/Hexan liefert 6-Fluor-3-(2-fluorphenyl)-1H-indol-2-carbonsäure-methylester. Eine Analysenprobe wird gereinigt, indem man mit Methylenchlorid als Lösungsmittel über Silicagel filtriert. Kristallisation aus Methylenchlorid/Hexan liefert farblose Kristalle, Smp. 158-160°.

## Beispiel 12

6-Fluor-3-(2-fluorphenyl)-1H-indol-2-carbonsäure

Ein Gemisch von 50 g (0,174 Mol) 6-Fluor-3-(2-fluorphenyl)-1H-indol-2-carbonsäure-methylester, 39 g (0,7 Mol) Kaliumhydroxyd, 150 ml Wasser und 1,5 l Methanol wird während 2 Stunden zum Rückfluss erhitzt. Danach wird das Gemisch mit Eisessig angesäuert und partiell eingedampft. Die heisse Lösung wird mit Wasser verdünnt, wobei das Produkt nach dem Animpfen kristallisiert. Nach dem Abkühlen werden die Kristalle abgetrennt und trocken gesaugt. Umkristallisation aus Aether/Hexan liefert 6-Fluor-3-(2-fluorphenyl)--1H-indol-2-carbonsäure als ein farbloses Produkt, Smp. 215-218°. Eine Analysenprobe wird aus den gleichen Lösungsmitteln umkristallisiert und weist einen unveränderten Smp. auf.

## Beispiel 13

6-Fluor-3-(2-fluorphenyl)-1H-indol-2-carboxamid

34,8 g (0,127 Mol) 6-Fluor-3-(2-fluorphenyl)-1H-indol--2-carbonsäure wird in das entsprechende Säurechlorid übergeführt, indem man sie mit 37,1 g (0,178 Mol) Phosphorpentachlorid in 3 l Methylenchlorid während 1 Stunde bei Raumtemperatur rührt. Danach wird solange Ammoniak eingeleitet, bis das Reaktionsgemisch basisch reagiert. Danach gibt man Wasser zu und rührt 1 Stunde. Die organische Phase wird abgetrennt, getrocknet und eingedampft. Kristallisation des Rückstandes aus Aethylacetat/Hexan liefert 6-Fluor-3-(2-fluorphenyl)-1H-indol-2-carboxamid, Smp. 191-193°. Für die Analyse wird es aus den gleichen Lösungsmitteln umkristallisiert, wobei man farblose Kristalle erhält, Smp. 195-197°.

## Beispiel 14

6-Fluor-3-(2-fluorphenyl)-1H-indol-2-methanamin-hydrochlorid

Eine Lösung von 5 g (18,4 mMol) 6-Fluor-3-(2-fluorphenyl)-1H-indol-2-carboxamid in 100 ml Tetrahydrofuran wird zu einer Suspension von 5 g Lithiumaluminiumhydrid in 150 ml Aether gegeben. Dieses Reaktionsgemisch wird unter Stickstoff während 6 Stunden zum Rückfluss erhitzt und danach über Nacht bei Raumtemperatur gerührt. Das überschüssige Reagens wird vorsichtig durch Zugabe von 25 ml Wasser zersetzt. Nach Verdünnen mit Aether wird das anorganische Material abfiltriert und mit Methylenchlorid gewaschen. Das Filtrat wird getrocknet und eingedampft. Der Rückstand wird in Aether gelöst und mit äthanolischer Salzsäure versetzt. Die ausgefallenen Kristalle werden abgetrennt und mit Aether gewaschen, wobei man 6-Fluor-3-(2-fluorphenyl)-1H--indol-2-methanamin-hydrochlorid als ein farbloses Produkt erhält. Die Analysenprobe wird aus Aethanol/Aether umkristallisiert und schmilzt bei 241-245° (Zers.).

## Beispiel 15

8-Fluor-5-(2-fluorphenyl)-1,3-dihydro-2H-1,4-benzodiazepin-2-on

Ein Gemisch von 8,4 g (28,5 mMol) 6-Fluor-3-(2-fluorphenyl)-1H-indol-2-methanamin-hydrochlorid, 84 ml Essigsäure, 8,4 ml Wasser und 8,4 g Chromtrioxyd wird in einem Kühlbad während 2 Stunden gerührt. Danach wird es auf Eiswasser gegossen, mit Ammoniak alkalisch gestellt und mit Methylenchlorid extrahiert. Die Extrakte werden getrocknet und nach Zugabe von 10 ml Essigsäure über das Wochenende stehengelassen. Die Methylenchloridlösung wird mit wässriger Bicarbonatlösung gewaschen, getrocknet und eingedampft. Der Rückstand wird unter Verwendung von Methylenchlorid/-Aethylacetat 1:1 (V/V) als Eluierungsmittel an 100 g Silicagel chromatographiert. Die Fraktionen, welche das Endprodukt enthalten, werden vereinigt und der Rückstand aus Aethylacetat/Hexan umkristallisiert, wobei man 8-Fluor-5--(2-fluorphenyl)-1,3-dihydro-2H-1,4-benzodiazepin-2-on als leicht gelbliche Kristalle erhält, Smp. 175-177°. Für die Analyse wird aus den gleichen Lösungsmitteln umkristallisiert, wobei man farblose Kristalle erhält, Smp. 177-179°.

## Beispiel 16

6-(2-Chlorphenyl)-9-fluor-4H-imidazo[1,5-a][1,4]benzodiazepin-3-carbonsäure-äthylester

In ähnlicher Weise wie in Beispiel 9 beschrieben, wird diese Verbindung durch Umsetzen von 5-(2-Chlorphenyl)-8--fluor-1,3-dihydro-2H-1,4-benzodiazepin-2-on mit Kalium-t--butoxyd und Diäthylchlorphosphat und nachfolgende Reaktion mit dem Anion von Aethylisocyanacetat erhalten. Der Ester wird aus Aether kristallisiert und aus Aethylacetat zu Analysenzwecken umkristallisiert. Die farblosen Kristalle schmelzen bei 213-215°.

Das Ausgangsmaterial für das Beispiel 16 wird durch Indoloxidation wie nachfolgend beschrieben hergestellt (Beispiele 17-21):

## Beispiel 17

3-(2-Chlorphenyl)-6-fluor-1H-indol-2-carbonsäure-äthylester

Diese Verbindung wird wie in Beispiel 11 beschrieben hergestellt, indem man das Diazoniumsalz von 3-Fluoranilin mit dem Natriumsalz von 2-Acetyl-3-(2-chlorphenyl)propionsäure-äthylester kuppelt und anschliessend in äthanolischer Salzsäure erhitzt. 3-(2-Chlorphenyl)-6-fluor-1H-indol-2--carbonsäure-äthylester wird erhalten und aus Aethanol/-Wasser kristallisiert. Eine Analysenprobe wird aus Hexan umkristallisiert, wobei man farblose Kristalle erhält, Smp. 123-125°.

## Beispiel 18

3-(2-Chlorphenyl)-6-fluor-1H-indol-2-carbonsäure

Diese Verbindung wird durch alkalische Hydrolyse des Aethylesters in ähnlicher Weise erhalten wie in Beispiel 12 beschrieben. Sie wird aus Aether/Hexan kristallisiert und aus dem leichen Lösungsmittel zu Analysenzwecken umkristallisiert, wobei man 3-(2-Chlorphenyl)-6-fluor-1H-indol-2--carbonsäure als leicht gelbliche Kristalle erhält, Smp. 198-200°.

## Beispiel 19

3-(2-Chlorphenyl)-6-fluor-1H-indol-2-carboxamid

3-(2-Chlorphenyl)-6-fluor-1H-indol-2-carbonsäure wird nach der Phosphorpentachlorid/Ammoniak-Methode wie in Beispiel 13 beschrieben in 3-(2-Chlorphenyl)-6-fluor-1H-indol-2-carboxamid überführt. Dieses wird aus Aethylacetat/-Hexan kristallisiert und zu Analysenzwecken aus den gleichen Lösungsmitteln umkristallisiert, wobei man farblose

Kristalle erhält, Smp. 208-211°.

## Beispiel 20

3-(2-Chlorphenyl)-6-fluor-1H-indol-2-methanamin-hydrochlorid

Eine Lösung von 11 g (38 mMol) 3-(2-Chlorphenyl)-6-fluor-1H-indol-2-carboxamid in 250 ml Tetrahydrofuran wird zu einer Suspension von 6,5 g Lithiumaluminiumhydrid in 500 ml Tetrahydrofuran gegeben. Nach 1-stündigem Erhitzen zum Rückfluss wird das überschüssige Reagens vorsichtig durch Zugabe von 40 ml Wasser zersetzt. Das anorganische Material wird abfiltriert, und das Filtrat getrocknet und eingedampft. Der Rückstand wird mit äthanolischer Salzsäure und Aether versetzt. Das ausgefallene Produkt wird abfiltriert, wobei man 3-(2-Chlorphenyl)-6-fluor-1H-indol-2-methanamin-hydrochlorid als Kristalle erhält, Smp. 242-245°. Die Analysenprobe wird aus Aethanol/Aether umkristallisiert und schmilzt bei 252-255°.

## Beispiel 21

5-(2-Chlorphenyl)-8-fluor-1,3-dihydro-2H-1,4-benzodiazepin-2-on

Diese Verbindung wird wie in Beispiel 15 hergestellt, indem man 3-(2-Chlorphenyl)-6-fluor-1H-indol-2-methanamin-hydrochlorid mit Chromtrioxyd oxidiert. Durch Filtrieren über Silicagel unter Verwendung von Methylenchlorid/Aethylacetat 1:1 (V/V) und Kristallisation aus Aethylacetat/Hexan wird das Produkt gereinigt und liefert farblose Kristalle, Smp. 239-242°.

## Beispiel 22

6-(2-Chlorphenyl)-4H-imidazo[1,5-a][1,4]benzodiazepin-3-carbonsäure

Eine Lösung von 13,8 g (35 mMol) 6-(2-Chlorphenyl)-4H-imidazo[1,5-a][1,4]benzodiazepin-3-carbonsäure-(1,1-dimethyl)

äthylester in 50 ml Trifluoressigsäure wird bei Raumtemperatur 16 Stunden gerührt. Das Reagens wird abgedampft, am Ende azeotrop mit Toluol. Der Rückstand wird mit Aethylacetat und Wasser gerührt, und die ausgefallenen Kristalle abgetrennt, wobei man 6-(2-Chlorphenyl)-4H-imidazo[1,5-a]-[1,4]benzodiazepin-3-carbonsäure erhält, Smp. 257-259°. Das Filtrat wird mit verdünntem Ammoniak extrahiert, und die Extrakte mit Essigsäure angesäuert. Der Niederschlag wird abgetrennt, wobei man eine zweite Fraktion erhält, welche aus Methanol/Aethylacetat umkristallisiert wird. Die Analysenprobe wird aus den gleichen Lösungsmitteln umkristallisiert und schmilzt bei 258-260°.

## Beispiel 23

9-Chlor-6-(2-chlorphenyl)-4H-imidazo[1,5-a][1,4]benzodiazepin-3-carbonsäure

Eine Lösung von 3,2 g (7,5 mMol) 9-Chlor-6-(2-chlorphenyl)-4H-imidazo[1,5-a][1,4]benzodiazepin-3-carbonsäure-(1,1-dimethyl)äthylester in 30 ml Trifluoressigsäure wird 1,5 Stunden bei Raumtemperatur stehengelassen. Das Reagens wird unter vermindertem Druck abgedampft, am Ende azeotrop mit Toluol. Der Rückstand wird in Methylenchlorid gelöst, mit Wasser gewaschen, getrocknet und eingedampft. Kristallisation aus Aethanol liefert 9-Chlor-6-(2-chlorphenyl)-4H-imidazo[1,5-a][1,4]benzodiazepin-3-carbonsäure als farbloses Produkt. Zu Analysenzwecken wird es aus Aethanol umkristalisiert, Smp. 264-265°.

## Beispiel 24

6-(2-Chlorphenyl)-9-fluor-4H-imidazo[1,5-a][1,4]benzodiazepin-3-carbonsäure

Eine Lösung von 6,3 g (16,5 mMol) 6-(2-Chlorphenyl)-9-fluor-4H-imidazo[1,5-a][1,4]benzodiazepin-3-carbonsäure-äthylester in 200 ml 6N Salzsäure wird 8 Stunden auf einem Dampfbad erhitzt. Nach dem Eindampfen zur Trockene wird der

Rückstand mit 150 ml Wasser, 6 g Natriumacetat und 6 ml Essigsäure 1 Stunde bei Raumtemperatur gerührt. Die ausgefallenen Kristalle werden abfiltriert, mit Wasser gewaschen, trocken gesaugt und aus Methanol/Aethylacetat umkristallisiert, wobei man 6-(2-Chlorphenyl)-9-fluor-4H-imidazo[1,5-a][1,4]benzodiazepin-3-carbonsäure als farbloses Produkt erhält, Smp. 270-272°.

## Beispiel 25

9-Fluor-6-(2-fluorphenyl)-4H-imidazo[1,5-a][1,4]benzodiazepin-3-carbonsäure

Eine Lösung von 2,6 g (7 mMol) 9-Fluor-6-(2-fluorphenyl)-4H-imidazo[1,5-a][1,4]benzodiazepin-3-carbonsäure--äthylester in 100 ml 6N Salzsäure wird 8 Stunden auf einem Dampfbad erhitzt. Nach dem Eindampfen zur Trockene wird der Rückstand mit 50 ml Wasser, 2 g Natriumacetat und 2 ml Essigsäure gerührt. Die ausgefallene Säure wird abfiltriert, mit Wasser gewaschen und trocken gesaugt. Danach wird sie aus Methanol/Aethylacetat umkristallisiert, wobei man 9-Fluor-6-(2-fluorphenyl)-4H-imidazo[1,5-a][1,4]benzodiazepin-3-carbonsäure erhält, Smp. 255-257° (Zers.).

## Beispiel 26

6-(2-Fluorphenyl)-4H-imidazo[1,5-a][1,4]benzodiazepin-3-carbonsäure

Eine Lösung von 16,9 g (45 mMol) 6-(2-Fluorphenyl)-4H--imidazo[1,5-a][1,4]benzodiazepin-3-carbonsäure-(1,1-dimethyl)äthylester in 60 ml Trifluoressigsäure wird bei Raumtemperatur 1,5 Stunden stehengelassen. Das Reagens wird unter vermindertem Druck abgedampft, und der Rückstand in 250 ml Methylenchlorid gelöst und mit 10%iger wässriger Natriumacetatlösung versetzt. Nachdem man 30 Minuten heftig gerührt hat, werden die ausgefallenen Kristalle abfiltriert und trocken gesaugt. Nach dem Trocknen unter vermindertem Druck über Nacht wird das Produkt aus Methanol/Aethylacetat

umkristallisiert, wobei man 6-(2-Fluorphenyl)-4H-imidazo-[1,5-a][1,4]benzodiazepin-3-carbonsäure als farblose Kristalle erhält, Smp. 241-243°.

## Beispiel 27

6-(2-Chlorphenyl)-4H-imidazo[1,5-a][1,4]benzodiazepin-3-carboxamid

(A) Ein Gemisch von 5 g (13,7 mMol) 6-(2-Chlorphenyl)-4H-imidazo[1,5-a][1,4]benzodiazepin-3-carbonsäure-äthylester, 5 g Ammoniumchlorid und 100 ml Methanol enthaltend 20% (V/V) Ammoniak wird in einer Stahlbombe 18 Stunden auf 100° erhitzt. Der Grossteil des Lösungsmittels wird eingedampft und der Rückstand mit Wasser verdünnt. Die Kristalle werden abfiltriert, trocken gesaugt und aus Methylenchlorid/Aethanol umkristallisiert, wobei man 6-(2-Chlorphenyl)-4H-imidazo[1,5-a][1,4]benzodiazepin-3-carboxamid als farblose Kristalle erhält, Smp. 310-314°.

(B) Dieselbe Verbindung wird erhalten, indem man 6-(2-Chlorphenyl)-4H-imidazo[1,5-a][1,4]benzodiazepin-3-carbonsäure mit Phosphorpentachlorid und das nicht isolierte Säurechlorid anschliessend mit Ammoniak umsetzt.

## Beispiel 28

6-(2-Fluorphenyl)-4H-imidazo[1,5-a][1,4]benzodiazepin-3-carboxamid

(A) Ein Gemisch von 1,5 g (4,3 mMol) 6-(2-Fluorphenyl)-4H-imidazo[1,5-a][1,4]benzodiazepin-3-carbonsäure-äthylester, 1,5 g Ammoniumchlorid und 20 ml methanolischem Ammoniak (20%, V/V) wird während 18 Stunden in einer Stahlbombe auf 100° erhitzt. Das übliche Aufarbeiten und Kristallisieren aus Methanol/Aethanol/Methylenchlorid liefert 6-(2-Fluorphenyl)-4H-imidazo[1,5-a][1,4]benzodiazepin-3-carboxamid als farblose Kristalle, Smp. >300°.

(B) Dieselbe Verbindung wird erhalten, indem man 6-(2-Fluorphenyl)-4H-imidazo[1,5-a][1,4]benzodiazepin-3-carbonsäure mit Phosphorpentachlorid und das nicht isolierte Säurechlorid anschliessend mit Ammoniak umsetzt.

## Beispiel 29

6-(2-Bromphenyl)-4H-imidazo[1,5-a][1,4]benzodiazepin-3-carboxamid

3 g (7,3 mMol) 6-(2-Bromphenyl)-4H-imidazo[1,5-a][1,4]-benzodiazepin-3-carbonsäure-äthylester wird auf einem Dampfbad während 8 Stunden mit 100 ml 6N Salzsäure erhitzt. Nach dem Eindampfen unter vermindertem Druck wird der Rückstand mit 150 ml Wasser, 2 ml Essigsäure und 1,5 g Natriumacetat während 1 Stunde bei Raumtemperatur gerührt. Das ausgefallene Produkt wird abfiltriert und trocken gesaugt. Umkristallisation aus Methanol/Aethylacetat liefert eine farblose Säure, welche bei 265-268° schmilzt. Diese Säure wird nach der Phosphorpentachlorid/Ammoniak-Methode wie sie in Beispiel 31 beschrieben ist, in das Amid übergeführt und liefert 6-(2-Bromphenyl)-4H-imidazo[1,5-a][1,4]benzodiazepin-3-carboxamid als Kristalle, welche bei 307-309° schmelzen (aus Methylenchlorid/Aethanol).

## Beispiel 30

6-[2-(Trifluormethyl)phenyl]-4H-imidazo[1,5-a][1,4]benzodiazepin-3-carboxamid

6-[2-(Trifluormethyl)phenyl]-4H-imidazo[1,5-a][1,4]benzodiazepin-3-carbonsäure-äthylester wird wie in Beispiel 27 beschrieben durch Erhitzen in methanolischem Ammoniak in Gegenwart von Ammoniumchlorid in 6-[2-(Trifluormethyl)-phenyl]-4H-imidazo[1,5-a][1,4]benzodiazepin-3-carboxamid überführt. Dieses wird aus Methylenchlorid/Aethanol umkristallisiert und liefert farblose Kristalle, Smp. 295-296°.

## Beispiel 31

9-Chlor-6-(2-chlorphenyl)-4H-imidazo[1,5-a][1,4]benzodiazepin-3-carboxamid

Ein Gemisch von 2,3 g (6 mMol) 9-Chlor-6-(2-chlorphenyl)-4H-imidazo[1,5-a][1,4]benzodiazepin-3-carbonsäure, 1,6 g (7,6 mMol) Phosphorpentachlorid und 75 ml Methylenchlorid wird 1,5 Stunden bei Raumtemperatur gerührt. Danach lässt man in die Lösung solange Ammoniak einleiten, bis diese basisch reagiert. Nach Zugabe von Wasser wird das Zweiphasensystem 1 Stunde gerührt. Das Produkt wird mit Methylenchlorid, welches 10% (V/V) Aethanol enthält, extrahiert. Die Extrakte werden getrocknet und eingedampft. Kristallisation aus Methylenchlorid/Aethanol liefert 9-Chlor-6-(2-chlorphenyl)-4H-imidazo[1,5-a][1,4]benzodiazepin-3-carboxamid als farblose Kristalle, Smp. 278-280°.

## Beispiel 32

9-Fluor-6-(2-fluorphenyl)-4H-imidazo[1,5-a][1,4]benzodiazepin-3-carboxamid

(A) 2,4 g 9-Fluor-6-(2-fluorphenyl)-4H-imidazo[1,5-a][1,4]-benzodiazepin-3-carbonsäure wird in das Amid übergeführt, wobei man nach der Phosphorpentachlorid/Ammoniak-Methode wie sie in Beispiel 31 beschrieben ist verfährt und das Endprodukt erhält, welches aus Methylenchlorid/Aethanol kristallisiert. Zu Analysenzwecken wird dieses aus den gleichen Lösungsmitteln umkristallisiert, wobei man 9-Fluor-6-(2-fluorphenyl)-4H-imidazo[1,5-a][1,4]benzodiazepin-3-carboxamid als farblose Kristalle erhält, Smp. 283-286°.

(B) Dieses Amid wird auch durch Umsetzen des entsprechenden Aethylesters mit Ammoniak/Ammoniumchlorid erhalten.

Beispiel 33

6-(2-Chlorphenyl)-9-fluor-4H-imidazo[1,5-a][1,4]benzodiazepin-3-carboxamid

3,3 g 6-(2-Chlorphenyl)-9-fluor-4H-imidazo[1,5-a][1,4]-benzodiazepin-3-carbonsäure wird nach der Phosphorpentachlorid/Ammoniak-Methode wie sie in Beispiel 31 beschrieben ist in das Amid übergeführt, wobei man nach Kristallisation aus Methylenchlorid/Aethanol das Endprodukt erhält, Smp. 295-297°.

Beispiel 34

6-(2-Chlorphenyl)-N-methyl-4H-imidazo[1,5-a][1,4]benzodiazepin-3-carboxamid

Umsetzen von 2 g (6 mMol) 6-(2-Chlorphenyl)-4H-imidazo[1,5-a][1,4]benzodiazepin-3-carbonsäure in 400 ml Methylenchlorid mit 1,7 g (8 mMol) Phosphorpentachlorid und nachfolgende Umsetzung mit Methylamin liefert das rohe Amid, welches über Silicagel filtriert wird, wobei man 5% (V/V) Aethanol in Methylenchlorid als Lösungsmittel verwendet. Kristallisation des eingedampften Filtrates aus Aethylacetat/Hexan liefert 6-(2-Chlorphenyl)-N-methyl-4H--imidazo[1,5-a][1,4]benzodiazepin-3-carboxamid als farblose Kristalle, Smp. 216-219°.

Beispiel 35

6-(2-Chlorphenyl)-N-äthyl-4H-imidazo[1,5-a][1,4]benzodiazepin-3-carboxamid

2 g 6-(2-Chlorphenyl)-4H-imidazo[1,5-a][1,4]benzodiazepin-3-carbonsäure werden wie in Beispiel 34 beschrieben in das Chlorid übergeführt und danach mit Aethylamin umgesetzt. Das Produkt wird durch Chromatographie an 50 g Silicagel unter Verwendung von Methylenchlorid/Aethylacetat 1:1 (V/V) gereinigt. Die reinen eingedampften Fraktionen werden aus Aethanol umkristallisiert, wobei man 6-(2-Chlorphenyl)-N-äthyl-4H-imidazo[1,5-a][1,4]benzodiazepin-3-carboxamid als farblose Kristalle erhält, Smp. 211-212°.

## Beispiel 36

6-(2-Chlorphenyl)-N-(1-methyläthyl)-4H-imidazo[1,5-a][1,4]-benzodiazepin-3-carboxamid

Umsetzen von 1,5 g (4,45 mMol) 6-(2-Chlorphenyl)-4H-imidazo[1,5-a][1,4]benzodiazepin-3-carbonsäure mit 1,3 g (6,25 mMol) Phosphorpentachlorid in 150 ml Methylenchlorid und nachfolgendes Umsetzen mit überschüssigem Isopropylamin liefert 6-(2-Chlorphenyl)-N-(1-methyläthyl)-4H-imidazo-[1,5-a][1,4]benzodiazepin-3-carboxamid. Eine Analysenprobe wird gereinigt, indem mit Aethylacetat/Methylenchlorid 1:1 (V/V) über Silicagel filtriert und aus Aethylacetat/Aether kristallisiert wird, wobei man farblose Kristalle erhält, Smp. 218-221°.

## Beispiel 37

9-Chlor-6-(2-chlorphenyl)-N-äthyl-4H-imidazo[1,5-a][1,4]-benzodiazepin-3-carboxamid

9-Chlor-6-(2-chlorphenyl)-4H-imidazo[1,5-a][1,4]benzodiazepin-3-carbonsäure wird wie in Beispiel 31 beschrieben in das Säurechlorid übergeführt und in situ mit überschüssigem Aethylamin umgesetzt. Kristallisation aus Methylenchlorid/Aethanol liefert 9-Chlor-6-(2-chlorphenyl)-N-äthyl-4H-imidazo[1,5-a][1,4]benzodiazepin-3-carboxamid als farbloses Produkt, Smp. 198-200°.

## Beispiel 38

(2-Amino-4-chlorphenyl)(2-chlorphenyl)methanon

52,8 ml (0,5 Mol) 3-Chloranilin wird in Portionen zu 142,5 ml (1,125 Mol) auf 120° erhitztes 2-Chlorbenzoylchlorid gegeben. Das Gemisch wird auf 180-200° erhitzt und dann portionenweise mit 87,2 g (0,64 Mol) Zinkchlorid versetzt.

Nach beendeter Zugabe wird das Reaktionsgemisch 1,5 Stunden auf 200-205° erhitzt. Nach dieser Zeit hat die Chlorwasserstoffentwicklung aufgehört. Nach Abkühlen auf 120° werden 500 ml 3N Salzsäure zugegeben, und das Gemisch zum Rückfluss erhitzt. Die Salzsäure wird danach abdekantiert, wobei dieses Prozedere noch zweimal wiederholt wird. Der Rückstand wird unter Erwärmen in 500 ml Essigsäure gelöst. Zu dieser Lösung werden 300 ml konz. Salzsäure gegeben, und das Gemisch während 18 Stunden zum Rückfluss erhitzt. Die Lösungsmittel werden unter vermindertem Druck entfernt, und der Rückstand zwischen Methylenchlorid und Wasser verteilt. Die organische Phase wird mit 3N Natriumhydroxydlösung gewaschen, getrocknet und eingedampft. Der Rückstand wird mit Toluol durch 300 g Silicagel filtriert. Kristallisation der das Endprodukt enthaltenden Fraktionen aus Aethanol liefert (2-Amino-4-chlorphenyl)(2-chlorphenyl)methanon als gelbe Kristalle in zwei Fraktionen. Eine Analysenprobe wird aus Aethanol umkristallisiert, Smp. 104-106°.

## Beispiel 39

2-Chlormethyl-4-(2-chlorphenyl)-6-fluor-1,2-dihydrochinazolin-3-oxid

Ein Gemisch von 25 g (0,1 Mol) (2-Amino-5-fluorphenyl)-(2-chlorphenyl)methanon, 9 g Hydroxylaminsulfat, 120 ml Aethanol und einer Lösung von 2-Chloracetaldehyd, welche durch 10-minütiges Erhitzen auf Rückfluss eines Gemisches von 20 g Chloracetaldehyd-dimethylacetal und 20 ml 1,5N Salzsäure hergestellt wurde, wird 3 Stunden bei Raumtemperatur gerührt. Danach wird es zwischen Wasser und Methylenchlorid verteilt. Die organische Phase wird mit gesättigter Natriumbicarbonatlösung gewaschen, getrocknet und eingedampft. Kristallisation des Rückstandes aus Aether/Hexan liefert 2-Chlormethyl-4-(2-chlorphenyl)-6-fluor-1,2-dihydrochinazolin-3-oxid in zwei Fraktionen. Die Analysenprobe wird aus Aethylacetat/Aether/Hexan umkristallisiert, wobei

man gelbe Kristalle erhält, Smp. 138-141°.

Beispiel 40

7-Chlor-2-chlormethyl-4-(2-chlorphenyl)-1,2-dihydrochinazolin-3-oxid

Diese Verbindung wird in 78%iger Ausbeute erhalten, indem man wie in Beispiel 41 beschrieben (2-Amino-4-chlorphenyl)(2-chlorphenyl)methanon mit Hydroxylaminsulfat und 2-Chloracetaldehyd umsetzt. Die Analysenprobe wird aus Methylenchlorid/Aethanol umkristallisiert, wobei man gelbliche Kristalle erhält, Smp. 197-198°. In diesem Fall wird das 1,2-Dihydroderivat vor Durchführung der Oxydation gemäss Beispiel 42 zu Analysenzwecken isoliert.

Beispiel 41

2-Chlormethyl-4-(2-chlorphenyl)chinazolin-3-oxid

Eine Lösung von 2-Chloracetaldehyd, welche durch 5-minütiges Erhitzen auf Rückfluss von 200 g 2-Chlor-1,1-dimethoxyäthan in 200 ml 1,5N Salzsäure erhalten wurde, wird zu einem Gemisch von 231 g (1 Mol) (2-Aminophenyl)(2-chlorphenyl)methanon, 90 g Hydroxylaminsulfat und 1,1 l Aethanol gegeben. Nach 2-stündigem Rühren bei Raumtemperatur wird das Reaktionsgemisch mit Wasser verdünnt und die ausgefallenen Kristalle abfiltriert, mit Wasser gewaschen und trocken gesaugt, wobei man rohes 2-Chlormethyl-4-(2-chlorphenyl)-1,2-dihydrochinazolin-3-oxid erhält, welches wie folgt direkt oxidiert wird: Eine Lösung des erhaltenen Materials in 1,4 l Methylenchlorid wird 2 Stunden mit 600 g Mangandioxid gerührt. Das Mangandioxid wird abfiltriert, und das Filtrat eingeengt und durch Zugabe von Aether kristallisiert, wobei man 2-Chlormethyl-4-(2-chlorphenyl)chinazolin-3-oxid als gelbe Kristalle erhält. Zu Analysenzwecken wird aus Methylenchlorid/Aether/Hexan umkristallisiert, Smp. 167-168°.

## Beispiel 42

7-Chlor-2-chlormethyl-4-(2-chlorphenyl)chinazolin-3-oxid

Oxidation von 24 g (70 mMol) 7-Chlor-2-chlormethyl-4--(2-chlorphenyl)-1,2-dihydrochinazolin-3-oxid in 300 ml Methylenchlorid mit 50 g Mangandioxid liefert 7-Chlor-2--chlormethyl-4-(2-chlorphenyl)chinazolin-3-oxid, Smp. 159-162°. Die Analysenprobe wird aus Tetrahydrofuran/Aethanol umkristallisiert, wobei man leicht gelbe Kristalle erhält, Smp. 161-162°.

## Beispiel 43

5-(2-Chlorphenyl)-1,3-dihydro-2H-1,4-benzodiazepin-2-ylidenessigsäure-äthylester-4-oxid

Eine Lösung von 193 ml 1,55 molarer (0,3 Mol) Lösung von Butyllithium in Hexan wird bei -50° zu 200 ml einer 2 molaren Lösung von Diisopropylamin in Tetrahydrofuran gegeben. Danach gibt man langsam Aethylacetat zu, wobei man die Temperatur unterhalb von -45° hält. Nach der Zugabe wird eine Lösung von 30,5 g (0,1 Mol) 2-Chlormethyl-4-(2--chlorphenyl)chinazolin-3-oxid in 200 ml Tetrahydrofuran dazugegeben. Nach Entfernen der Kühlung wird das Reaktionsgemisch 2 Stunden unter Stickstoff gerührt. Dann wird es durch Zugabe von 30 ml Essigsäure angesäuert und zwischen Toluol und Wasser verteilt. Die organische Phase wird getrocknet und eingedampft. Kristallisation des Rückstandes aus Aether liefert 5-(2-Chlorphenyl)-1,3-dihydro-2H-1,4--benzodiazepin-2-ylidenessigsäure-äthylester-4-oxid. Zu Analysenzwecken wird eine Probe davon aus Methylenchlorid/-Aether umkristallisiert, Smp. 172-174°.

## Beispiel 44

5-(2-Fluorphenyl)-1,3-dihydro-2H-1,4-benzodiazepin-2-ylidenessigsäure-(1,1-dimethyl)äthylester-4-oxid

Eine Lösung von 406 ml 1,6 molarem (0,65 Mol) n-Butyllithium in Hexan wird bei -50° zu 400 ml (0,8 Mol) einer 2 molaren Lösung von Diisopropylamin in Tetrahydrofuran gegeben. Nach 10-minütigem Rühren unter Stickstoff werden 108 ml (0,8 Mol) t-Butylacetat zugegeben, wobei man die Temperatur zwischen -40 und -50° hält. Nach der Zugabe wird das Reaktionsgemisch 10 Minuten gerührt, währenddem man auf -60° kühlt. Eine Lösung von 57,7 g (0,2 Mol) 2-Chlormethyl-4-(2-fluorphenyl)chinazolin-3-oxid in 750 ml Tetrahydrofuran wird dann auf einmal zugegeben. Das Kühlen wird beendet, und das Gemisch während 2,5 Stunden gerührt. Nach Ansäuern mit Eisessig wird das Reaktionsgemisch zwischen Wasser und Toluol verteilt. Die organische Phase wird getrocknet und eingeengt. Das bei der Verdünnung mit Hexan ausgefallene Produkt wird abgetrennt, und mit ein wenig Aether gewaschen, wobei man 5-(2-Fluorphenyl)-1,3-dihydro--2H-1,4-benzodiazepin-2-ylidenessigsäure-(1,1-dimethyl)äthylester-4-oxid erhält, Smp. 184-186°. Zu Analysenzwecken wird aus Aethanol umkristallisiert, wobei man farblose Kristalle erhält, Smp. 186-188° (Zers.).

## Beispiel 45

5-(2-Chlorphenyl)-1,3-dihydro-2H-1,4-benzodiazepin-2-ylidenessigsäure-(1,1-dimethyl)äthylester-4-oxid

Umsetzen von 61 g (0,2 Mol) 2-Chlormethyl-4-(2-chlorphenyl)chinazolin-3-oxid mit dem Anion von t-Butylacetat wie in Beispiel 44 beschrieben liefert 5-(2-Chlorphenyl)--1,3-dihydro-2H-1,4-benzodiazepin-2-ylidenessigsäure-(1,1-dimethyl)äthylester-4-oxid, Smp. 229-231° (Zers.). Die Analysenprobe wird aus Methylenchlorid/Aethanol umkristallisiert.

## Beispiel 46

8-Chlor-5-(2-chlorphenyl)-1,3-dihydro-2H-1,4-benzodiazepin-2-ylidenessigsäure-(1,1-dimethyl)äthylester-4-oxid

Umsetzen von 20 g (0,059 Mol) 7-chlor-2-chlormethyl-4--(2-chlorphenyl)chinazolin-3-oxid mit dem Anion von t-Butylacetat wie in Beispiel 44 beschrieben liefert 8-Chlor-5--(2-chlorphenyl)-1,3-dihydro-2H-1,4-benzodiazepin-2-ylidenessigsäure-(1,1-dimethyl)äthylester-4-oxid, welcher aus Aether kristallisiert. Für Analysenzwecke wird aus Aether umkristallisiert, Smp. 197-199°.

## Beispiel 47

5-(2-Chlorphenyl)-1,3-dihydro-2H-1,4-benzodiazepin-2-ylidenessigsäure-(1,1-dimethyl)äthylester

145 ml Phosphortrichlorid werden in drei Portionen innert 15 Minuten zu einer Lösung von 145 g (0,376 Mol) 5-(2-Chlorphenyl)-1,3-dihydro-2H-1,4-benzodiazepin-2-ylidenessigsäure-(1,1-dimethyl)äthylester-4-oxid in 1,5 l mit Eiswasser gekühltem Methylenchlorid gegeben. Nach weiterem 15-minütigem Rühren wird das Reaktionsgemisch unter vermindertem Druck eingedampft. Der Rückstand wird zwischen Methylenchlorid und 10%iger wässriger Natriumcarbonatlösung verteilt. Die organische Phase wird getrocknet und eingedampft, wobei man 5-(2-Chlorphenyl)-1,3-dihydro-2H-1,4--benzodiazepin-2-ylidenessigsäure-(1,1-dimethyl)äthylester als gelbes Oel erhält. Eine 2 g-Probe wird über Silicagel filtriert, wobei man 10% (V/V) Aethylacetat in Methylenchlorid als Lösungsmittel verwendet. Kristallisation aus Methylenchlorid/Aethanol liefert leicht gelbliche Kristalle, Smp. 170-173°.

## Beispiel 48

5-(2-Fluorphenyl)-1,3-dihydro-2H-1,4-benzodiazepin-2-ylidenessigsäure-(1,1-dimethyl)äthylester

126 ml Phosphortrichlorid werden in drei Portionen innert 15 Minuten zu einer Lösung von 126,5 g (0,343 Mol) 5-(2-Fluorphenyl)-1,3-dihydro-2H-1,4-benzodiazepin-2-ylidenessigsäure-(1,1-dimethyl)äthylester-4-oxid in 1,3 l mit Eiswasser gekühltem Methylenchlorid gegeben. Nach weiterem 30-minütigem Rühren wird die Lösung eingedampft und das

verbleibende Oel zwischen Methylenchlorid und 10%iger wässriger Natriumcarbonatlösung verteilt. Die organische Phase wird mit Carbonatlösung gewaschen, getrocknet und eingedampft. Kristallisation des Rückstandes aus Methanol liefert 5-(2-Fluorphenyl)-1,3-dihydro-2H-1,4-benzodiazepin-2-ylidenessigsäure-(1,1-dimethyl)äthylester als Kristalle, Smp. 154-157°. Die Analysenprobe wird aus Methanol umkristallisiert, Smp. 155-157°.

## Beispiel 49

8-Chlor-5-(2-chlorphenyl)-1,3-dihydro-2H-1,4-benzodiazepin-2-ylidenessigsäure-(1,1-dimethyl)äthylester

Diese Verbindung erhält man, indem man wie in Beispiel 47 beschrieben, 8-Chlor-5-(2-chlorphenyl)-1,3-dihydro-2H-1,4-benzodiazepin-2-ylidenessigsäure-(1,1-dimethyl)äthylester-4-oxid mit Phosphortrichlorid umsetzt. Kristallisation aus Aether und Umkristallisation zu Analysenzwecken aus Methylenchlorid/Aethanol liefert farblose Kristalle, Smp. 158-160°.

## Beispiel 50

5-(2-Chlorphenyl)-α-(hydroxyimino)-3H-1,4-benzodiazepin-2-essigsäure-(1,1-dimethyl)äthylester

53 ml Phosphortrichlorid werden in zwei Portionen innert 15 Minuten zu einer eisgekühlten Lösung von 53,4 g (0,138 Mol) 5-(2-Chlorphenyl)-1,3-dihydro-2H-1,4-benzodiazepin-2-ylidenessigsäure-(1,1-dimethyl)äthylester-4-oxid in 650 ml Methylenchlorid gegeben. Nach weiterem 15-minütigem Rühren wird das Lösungsmittel unter vermindertem Druck abgedampft, und der Rückstand zwischen Methylenchlorid und 10%iger wässriger Natriumcarbonatlösung verteilt. Nach dem Aufarbeiten wird der Rückstand mit 12,45 g (0,18 Mol) Natriumnitrit in 350 ml Eisessig versetzt. Verdünnen mit Wasser, Filtrieren und Umkristallisation aus Tetrahydrofuran/Aethanol liefert 5-(2-Chlorphenyl)-α-(hydroxy-

imino)-3H-1,4-benzodiazepin-2-essigsäure-(1,1-dimethyl)äthylester als leicht gelbliche Kristalle, Smp. 229-230°. Zu Analysenzwecken wird aus Methylenchlorid/Aethanol umkristallisiert, wobei der Schmelzpunkt unverändert bleibt.

## Beispiel 51

5-(2-Chlorphenyl)-α-(hydroxyimino)-3H-1,4-benzodiazepin-2-essigsäure-äthylester

25 ml Phosphortrichlorid werden in drei Portionen innert 15 Minuten zu einer Lösung von 25,2 g (0,07 Mol) 5-(2-Chlorphenyl)-1,3-dihydro-2H-1,4-benzodiazepin-2-ylidenessigsäure-äthylester-4-oxid in 250 ml in Eiswasser gekühltem Methylenchlorid gegeben. Nach weiterem 15-minütigem Rühren bei Raumtemperatur wird das Reaktionsgemisch eingedampft. Der Rückstand wird zwischen Methylenchlorid und 10%iger wässriger Natriumcarbonatlösung verteilt. Die organische Phase wird getrocknet und eingedampft, und der Rückstand in 150 ml Eisessig gelöst und mit 6,8 g Natriumnitrit in zwei Portionen versetzt. Das Rektionsgemisch wird während 1 Stunde gerührt und danach mit Wasser verdünnt. Das ausgefallene Produkt wird abfiltriert, mit Wasser gewaschen und trocken gesaugt. Umkristallisation aus Tetrahydrofuran/Aethanol liefert 5-(2-Chlorphenyl)-α-(hydroxyimino)--3H-1,4-benzodiazepin-2-essigsäure-äthylester als gelbliche Kristalle, Smp. 245-248° (Zers.).

## Beispiel 52

8-Chlor-5-(2-chlorphenyl)-α-(hydroxyimino)-3H-1,4-benzodiazepin-2-essigsäure-(1,1-dimethyl)äthylester

4 g (58 mMol) Natriumnitrit werden zu einer Lösung von 19 g (47 mMol) 8-Chlor-5-(2-chlorphenyl)-1,3-dihydro-2H--1,4-benzodiazepin-2-ylidenessigsäure-(1,1-dimethyl)äthylester in 150 ml Eisessig gegeben. Nach 30-minütigem Rühren bei Raumtemperatur wird das Reaktionsgemisch mit Wasser verdünnt, und die ausgefallenen Kristalle abfiltriert, mit

Wasser gewaschen und trocken gesaugt. Umkristallisation aus Methylenchlorid/Aethanol liefert 8-Chlor-5-(2-chlorphenyl)-α-(hydroxyimino)-3H-1,4-benzodiazepin-2-essigsäure-(1,1-dimethyl)äthylester, Smp. 208-210°.

## Beispiel 53

5-(2-Chlorphenyl)-α-(hydroxyimino)-3H-1,4-benzodiazepin-2-essigsäure-äthylester-4-oxid

Eine Suspension von 35,7 g (0,1 Mol) 5-(2-Chlorphenyl)-1,3-dihydro-2H-1,4-benzodiazepin-2-ylidenessigsäure-äthylester-4-oxid in 225 ml Eisessig wird mit 9,75 g (0,14 Mol) Natriumnitrit in zwei Portionen innert 10 Minuten versetzt. Nach 1-stündigem Rühren bei Raumtemperatur wird das kristalline Produkt durch Verdünnen mit Wasser weiter ausgefällt und abfiltriert. Es wird mit Wasser gewaschen und trocken gesaugt. Umkristallisation aus Tetrahydrofuran/Aethanol liefert 5-(2-Chlorphenyl)-α-(hydroxyimino)-3H-1,4-benzodiazepin-2-essigsäure-äthylester-4-oxid als gelbliche Kristalle, Smp. 262-264° (Zers.). Eine zweite Fraktion kann aus der Mutterlauge isoliert werden.

## Beispiel 54

5-(2-Fluorphenyl)-α-(hydroxyimino)-3H-1,4-benzodiazepin-2-essigsäure-(1,1-dimethyl)äthylester mit 1 Mol Aethanol solvatisiert

Umsetzen von 70,4 g (0,2 Mol) 5-(2-Fluorphenyl)-1,3-dihydro-2H-1,4-benzodiazepin-2-ylidenessigsäure-(1,1-dimethyl)-äthylester mit 19,3 g (0,28 Mol) Natriumnitrit in 425 ml Eisessig liefert 5-(2-Fluorphenyl)-α-(hydroxyimino)-3H-1,4-benzodiazepin-2-essigsäure-(1,1-dimethyl)äthylester als ein mit 1 Mol Aethanol solvatisiertes Produkt, Smp. 214-216° (Zers.). Zu Analysenzwecken wird es aus Methylenchlorid/Aethanol umkristallisiert.

## Beispiel 55

6-(2-Chlorphenyl)-4H-imidazo[1,5-a][1,4]benzodiazepin-3-carbonsäure-(1,1-dimethyl)äthylester

Ein Gemisch von 17 g (42,5 mMol) 5-(2-Chlorphenyl)--α-(hydroxyimino)-3H-1,4-benzodiazepin-2-essigsäure-(1,1-dimethyl)äthylester, 240 ml Tetrahydrofuran, 120 ml Methanol, 17 g Raney-nickel und 3 ml methanolischem Ammoniak (20%, V/V) wird bei Atmosphärendruck 2 Stunden hydriert. Der Katalysator wird abfiltriert, und das Filtrat unter vermindertem Druck eingedampft. Der Rückstand wird in 100 ml Aethylacetat gelöst. Die Lösung wird mit 6,8 ml (50 mMol) Dimethylformamid-dimethylacetal versetzt und 5 Minuten zum Rückfluss erhitzt. Nach Zugabe von 100 ml Hexan kristallisiert das Produkt beim Abkühlen aus. Man trennt ab, wobei man 6-(2-Chlorphenyl)-4H-imidazo[1,5-a][1,4]benzodiazepin--3-carbonsäure-(1,1-dimethyl)äthylester als bräunliche Kristalle erhält. Die Analysenprobe wird aus Methylenchlorid/-Aethylacetat umkristallisiert, wobei man farblose Kristalle erhält, Smp. 247-249°.

## Beispiel 56

6-(2-Fluorphenyl)-4H-imidazo[1,5-a][1,4]benzodiazepin-3-carbonsäure-(1,1-dimethyl)äthylester

38,2 g (0,09 Mol) 5-(2-Fluorphenyl)-α-(hydroxyimino)-3H-1,4-benzodiazepin-2-essigsäure-(1,1-dimethyl)äthylester, der mit 1 Mol Aethanol solvatisiert ist, wird wie in Beispiel 55 beschrieben während 3 Stunden über Raney-nickel hydriert. Das rohe Hydrierungsprodukt wird in 200 ml Aethylacetat gelöst. Nach Zugabe von 19 ml Dimethylformamid-dimethylacetal wird das Gemisch 5 Minuten zum Rückfluss erhitzt. Das Produkt kristallisiert bei der Zugabe von 500 ml Hexan und Abkühlen aus, wobei man 6-(2-Fluorphenyl)-4H-imidazo[1,5-a][1,4]benzodiazepin-2-essigsäure-(1,1-dimethyl)äthylester als Kristalle erhält, Smp. 216-219°. Die Analysenprobe wird aus Aethylacetat/Hexan umkristallisiert, Smp. 218-220°.

## Beispiel 57

<u>6-(2-Chlorphenyl)-4H-imidazo[1,5-a][1,4]benzodiazepin-3-carbonsäure-äthylester-5-oxid</u>

Ein Gemisch von 31 g (0,08 Mol) 5-(2-Chlorphenyl)--α-(hydroxyimino)-3H-1,4-benzodiazepin-2-essigsäure-äthylester-4-oxid, 600 ml Tetrahydrofuran, 300 ml Aethanol, 12 ml Methanol enthaltend 20% (V/V) Ammoniak und 31 g Raney-nickel wird bei Atmosphärendruck solange hydriert, bis die Wasserstoffaufnahme aufhört (2 Stunden). Nach Abfiltrieren und Eindampfen wird der Rückstand in 150 ml Aethylacetat gelöst. 16 ml Dimethylformamid-dimethylacetal werden zugegben, und die Lösung 5 Minuten zum Rückfluss erhitzt. Das Produkt kristallisiert beim Abkühlen aus und wird abfiltriert und mit Aether gewaschen, wobei man 6-(2--Chlorphenyl)-4H-imidazo[1,5-a][1,4]benzodiazepin-3-carbonsäure-äthylester-5-oxid als Kristalle erhält, Smp. 245-248°. Zu Analysenzwecken wird aus Methylenchlorid/Aethylacetat umkristallisiert, wobei der Schmelzpunkt unverändert bleibt.

## Beispiel 58

<u>6-(2-Chlorphenyl)-1-methyl-4H-imidazo[1,5-a][1,4]benzodiazepin-3-carbonsäure-(1,1-dimethyl)äthylester</u>

Ein Gemisch von 12 g (0,03 Mol) 5-(2-Chlorphenyl)--α-(hydroxyimino)-3H-1,4-benzodiazepin-2-essigsäure-(1,1-dimethyl)äthylester, 200 ml Tetrahydrofuran, 100 ml Aethanol, 3 ml methanolischem Ammoniak (20%, V/V) und 23 g Raney--nickel werden 1,5 Stunden bei Atmosphärendruck hydriert. Der Katalysator wird abfiltriert, und das Filtrat unter vermindertem Druck eingedampft. Der gelbe Rückstand wird in 400 ml Methylenchlorid gelöst und mit 5 ml (0,09 Mol) Acetaldehyd versetzt. Nach 20-minütigem Rühren bei Raumtemperatur werden 12 g Mangandioxid zugegeben und das Rühren 30 Minuten fortgeführt. Das Mangandioxid wird abfiltriert und das Filtrat eingedampft. Kristallisation des Rückstandes aus Aethylacetat liefert 6-(2-Chlorphenyl)-1--methyl-4H-imidazo[1,5-a][1,4]benzodiazepin-3-carbonsäure-(1,1-dimethyl)äthylester, Smp. 250-253°. Die Analysenprobe wird aus Aethylacetat umkristallisiert, wobei der Schmelzpunkt unverändert bleibt.

## Beispiel 59

6-(2-Chlorphenyl)-1-methyl-4H-imidazo[1,5-a][1,4]benzodiazepin-3-carbonsäure-äthylester-5-oxid

11 g 5-(2-Chlorphenyl)-α-(hydroxyimino)-3H-1,4-benzodiazepin-2-essigsäure-äthylester-4-oxid werden wie in Beispiel 58 beschrieben hydriert, und das rohe Hydrierungsprodukt in 350 ml Methylenchlorid gelöst. 5,5 ml Acetaldehyd werden zugegeben, und die Lösung 20 Minuten gerührt. Nach erfolgter Zugabe von 11 g Mangandioxid rührt man 30 Minuten weiter. Das Mangandioxid wird über Celit abfiltriert, und das Filtrat eingedampft. Kristallisation des Rückstandes aus Aethylacetat liefert 6-(2-Chlorphenyl)-1-methyl-4H-imidazo[1,5-a][1,4]benzodiazepin-3-carbonsäure-äthylester-5--oxid, Smp. 221-224°. Die Analysenprobe wird aus Methylenchlorid/Aethylacetat umkristallisiert, wobei man farblose Kristalle erhält, Smp. 224-226°.

## Beispiel 60

6-(2-Chlorphenyl)-1-äthyl-4H-imidazo[1,5-a][1,4]benzodiazepin-3-carbonsäure-(1,1-dimethyl)äthylester

Ein Gemisch von 5 g (12,6 mMol) 5-(2-Chlorphenyl)-α--(hydroxyimino)-3H-1,4-benzodiazepin-2-essigsäure-(1,1-dimethyl)äthylester, 100 ml Tetrahydrofuran, 50 ml Aethanol, 2 ml Methanol enthaltend 20% (V/V) Ammoniak und 5 g Raney--nickel wird bei Atmosphärendruck solange hydriert, bis die Wasserstoffabsorption aufhört (1-2 Stunden). Nach Filtration und Eindampfen wird der Rückstand in 200 ml Methylenchlorid gelöst und nach Zugabe von 2 ml Propionaldehyd

20 Minuten gerührt. Die Lösung wird danach mit 5 g Mangandioxid versetzt und weitere 20 Minuten gerührt. Das Mangandioxid wird über Celit abfiltriert. Das Filtrat wird eingedampft, und der Rückstand aus Aether umkristallisiert, wobei man 6-(2-Chlorphenyl)-1-äthyl-4H-imidazo[1,5-a][1,4]-benzodiazepin-3-carbonsäure-(1,1-dimethyl)äthylester erhält, welcher aus Aethylacetat/Hexan zu Analysenzwecken umkristallisiert wird, Smp. 211-213°.

## Beispiel 61

9-Chlor-6-(2-chlorphenyl)-4H-imidazo[1,5-a][1,4]benzodiazepin-3-carbonsäure-(1,1-dimethyl)äthylester

Ein Gemisch von 15 g (35 mMol) 8-chlor-5-(2-chlorphenyl)-α-(hydroxyimino)-3H-1,4-benzodiazepin-2-essigsäure-(1,1-dimethyl)äthylester, 150 ml Tetrahydrofuran, 75 ml Methanol, 15 g Raney-nickel und 5 ml methanolischem Ammoniak (20%, V/V) wird 2,5 Stunden bei Atmosphärendruck hydriert, nach welcher Zeit die Wasserstoffaufnahme aufgehört hat. Der Katalysator wird abfiltriert, und das Filtrat unter vermindertem Druck eingedampft. Der Rückstand wird in 200 ml Aethylacetat gelöst, und die Lösung mit 10 ml Dimethylformamid-dimethylacetal versetzt. Nach 5-minütigem Erhitzen zum Rückfluss wird das Lösungsmittel abgedampft, und der Rückstand aus Aether umkristallisiert, wobei man 9-Chlor-6-(2-chlorphenyl)-4H-imidazo[1,5-a][1,4]benzodiazepin-3-carbonsäure-(1,1-dimethyl)äthylester erhält, Smp. 232-234°. Die Analysenprobe wird durch Chromatographie an Silicagel unter Verwendung von Methylenchlorid/Aethylacetat 1:1 (V/V) und Kristallisation aus Aethanol gereinigt, Smp. 234-236°.

## Beispiel 62

6-(2-Chlorphenyl)-1-methyl-4H-imidazo[1,5-a][1,4]benzodiazepin-3-carbonsäure

Eine Lösung von 10 g (24,5 mMol) 6-(2-Chlorphenyl)-1--

methyl-4H-imidazo[1,5-a][1,4]benzodiazepin-3-carbonsäure (1,1-dimethyl)äthylester in 50 ml Trifluoressigsäure wird über Nacht stehengelassen, wobei man langsam Stickstoff durchbläst. Das Gemisch wird unter vermindertem Druck eingedampft, am Ende azeotrop mit Toluol. Der Rückstand wird zwischen verdünntem Ammoniak und Aethylacetat verteilt. Die Ammoniakextrakte werden mit Essigsäure angesäuert und teilweise eingedampft. Die ausgefallenen Kristalle werden abgetrennt und mit Wasser gewaschen, wobei man 6-(2-Chlorphenyl)-1-methyl-4H-imidazo[1,5-a][1,4]benzodiazepin-3-carbon säure als farblose Kristalle erhält. Umkristallisation aus Methanol/Aethylacetat liefert ein Produkt, welches bei 272-274° schmilzt.

## Beispiel 63

6-(2-Chlorphenyl)-1-methyl-4H-imidazo-[1,5-a][1,4]benzodiazepin-3-carboxamid

Umsetzen von 18,8 g (53 mMol) 6-(2-Chlorphenyl)-1-methyl-4H-imidazo[1,5-a][1,4]benzodiazepin-3-carbonsäure mit 15,6 g (75 mMol) Phosphorpentachlorid in 2 l Methylenchlorid und nachfolgendes Umsetzen mit überschüssigem Ammoniakgas führt zu 6-(2-Chlorphenyl)-1-methyl-4H-imidazo[1,5-a]-[1,4]benzodiazepin-3-carboxamid, welches aus Methylenchlorid/Aethanol umkristallisiert wird. Die Analysenprobe wird aus Tetrahydrofuran/Aethanol umkristallisiert, Smp. 318-321°.

## Beispiel 64

6-(2-Chlorphenyl)-N-äthyl-1-methyl-4H-imidazo[1,5-a][1,4]-benzodiazepin-3-carboxamid

Unter Verwendung der Phosphorpentachlorid/Aethylamin--Methode wie in Beispiel 63 beschrieben werden 2,8 g (8 mMol) 6-(2-Chlorphenyl)-1-methyl-4H-imidazo[1,5-a][1,4]-benzodiazepin-3-carbonsäure in das analoge Aethylamid überführt und aus Methylenchlorid/Aethylacetat kristallisiert.

Die Analysenprobe wird aus den gleichen Lösungsmitteln umkristallisiert, Smp. 240-242°.

Beispiel 65

6-(2-Chlorphenyl)-1-äthyl-4H-imidazo[1,5-a][1,4]benzodiazepin-3-carboxamid

Umsetzen von 1,85 g (5 mMol) 6-(2-Chlorphenyl)-1-äthyl-4H-imidazo[1,5-a][1,4]benzodiazepin-3-carbonsäure, welche durch Spaltung der t-Butylestergruppe im Endprodukt von Beispiel 60 mit Trifluoressigsäure erhalten wird, mit 1,47 g (7 mMol) Phosphorpentachlorid in 200 ml Methylenchlorid während 1 Stunde und nachfolgendes Umsetzen mit Ammoniak liefert 6-(2-Chlorphenyl)-1-äthyl-4H-imidazo-[1,5-a][1,4]benzodiazepin-3-carboxamid als farbloses Produkt, Smp. 288-290°, welches aus Tetrahydrofuran/Aethanol kristallisiert.

Beispiel 66

6-(2-Chlorphenyl)-1-methyl-4H-imidazo[1,5-a][1,4]benzodiazepin-3-carboxamid-5-oxid

Ein Gemisch von 1 g (2,5 mMol) 6-(2-Chlorphenyl)-4H-imidazo[1,5-a][1,4]benzodiazepin-3-carbonsäure-äthylester-5-oxid, 1 g Ammoniumchlorid und 20 ml Methanol enthaltend 20% (V/V) Ammoniak wird während 18 Stunden bei 95-100° in einer Stahlbombe erhitzt. Das Reaktionsgemisch wird mit Wasser verdünnt, und die ausgefallenen Kristalle abfiltriert, trocken gesaugt und aus Methylenchlorid/Methanol umkristallisiert, wobei man 6-(2-Chlorphenyl)-1-methyl-4H-imidazo-[1,5-a][1,4]benzodiazepin-3-carboxamid-5-oxid als farbloses Produkt erhält, Smp. 307-310° (Zers.).

## Beispiel 67

6-(2-Chlorphenyl)-4H-imidazo[1,5-a][1,4]benzodiazepin-3-carboxamid-5-oxid

(A) 1,55 g (9 mMol) m-Chlorperbenzoesäure werden zu einer Lösung von 2 g (5,9 mMol) 6-(2-Chlorphenyl)-4H-imidazo-[1,5-a][1,4]benzodiazepin-3-carboxamid in 500 ml Methylenchlorid gegeben. Nach Stehenlassen bei Raumtemperatur über Nacht wird die Lösung mit gesättigter wässriger Natriumbicarbonatlösung gewaschen, getrocknet und eingedampft. Der kristalline Rückstand wird aus Methylenchlorid/Methanol umkristallisiert, wobei man 6-(2-Chlorphenyl)-4H-imidazo-[1,5-a][1,4]benzodiazepin-3-carboxamid-5-oxid als farbloses Produkt erhält, Smp. 301-304° (Zers.).

(B) Ein Gemisch von 3 g (7,6 mMol) 6-(2-Chlorphenyl)-4H--imidazo[1,5-a][1,4]benzodiazepin-3-carbonsäure-äthylester-5--oxid, 3 g Ammoniumchlorid und 75 ml 20%igem methanolischem Ammoniak wird während 18 Stunden in einer Stahlbombe auf 100° erhitzt. Das Reaktionsgemisch wird mit Wasser verdünnt, und das Produkt abfiltriert, trocken gesaugt und aus Methylenchlorid/Methanol umkristallisiert, wobei man 6-(2--Chlorphenyl)-4H-imidazo[1,5-a][1,4]benzodiazepin-3-carboxamid-5-oxid als Kristalle erhält, welche mit dem in Paragraph (A) erhaltenen Produkt identisch sind.

## Beispiel 68

N-Acetyl-6-(2-chlorphenyl)-4H-imidazo[1,5-a][1,4]benzodiazepin-3-carboxamid

3,36 g (10 mMol) 6-(2-Chlorphenyl)-4H-imidazo[1,5-a]-[1,4]benzodiazepin-3-carboxamid werden in 125 ml Acetanhydrid 1,5 Stunden zum Rückfluss erhitzt, während man 50 ml des Reagens abdestilliert. Das Lösungsmittel wird abgedampft, am Ende azeotrop mit Xylol, und der Rückstand aus Aethylacetat kristallisiert, wobei man N-Acetyl-6-(2-chlorphenyl)-4H-imidazo[1,5-a][1,4]benzodiazepin-3-carboxamid

erhält, welches aus Tetrahydrofuran/Aethylacetat/Hexan umkristallisiert wird und farblose Kristalle liefert, Smp. 178-180°.

## Beispiel 69

N-Acetyl-6-(2-chlorphenyl)-4H-imidazo[1,5-a][1,4]benzodiazepin-3-carboxamid-5-oxid

1,57 g (9 mMol) m-Chlorperbenzoesäure werden zu einer Lösung von 2,3 g (6 mMol) N-Acetyl-6-(2-chlorphenyl)-4H-imidazo[1,5-a][1,4]benzodiazepin-3-carboxamid in 125 ml Methylenchlorid gegeben. Nach Stehenlassen bei Raumtemperatur während 24 Stunden wird die Lösung mit gesättigter Natriumbicarbonatlösung gewaschen, getrocknet und eingedampft. Kristallisation des Rückstandes aus Methylenchlorid/Methanol liefert N-Acetyl-6-(2-chlorphenyl)-4H-imidazo[1,5-a][1,4]benzodiazepin-3-carboxamid-5-oxid als ein farbloses Produkt, Smp. 275-278° (Zers.).

## Beispiel 70

4-Acetyloxy-N-acetyl-6-(2-chlorphenyl)-4H-imidazo[1,5-a]-[1,4]benzodiazepin-3-carboxamid

Eine Lösung von 1,8 g (4,5 mMol) N-Acetyl-6-(2-chlorphenyl)-4H-imidazo[1,5-a][1,4]benzodiazepin-3-carboxamid-5-oxid in 150 ml Essigsäureanhydrid wird 1,5 Stunden zum Rückfluss erhitzt. Die Lösung wird eingedampft, am Ende azeotrop mit Xylol. Der Rückstand wird aus Aethylacetat/Aether kristallisiert, wobei man 4-Acetyloxy-N-acetyl-6-(2-chlorphenyl)-4H-imidazo[1,5-a][1,4]benzodiazepine-3-carboxamid als Rohprodukt erhält. Dieses wird gereinigt, indem man es über Silicagel mit 10% (V/V) Aethylacetat in Methylenchlorid filtriert. Eindampfen und Kristallisation aus Aethylacetat/Hexan liefert farblose Kristalle, die bei 170-173° schmelzen.

## Beispiel 71

N-Acetyl-4-acetyloxy-6-(2-chlorphenyl)-1-methyl-4H-imidazo-[1,5-a][1,4]benzodiazepin-3-carboxamid

Eine Suspension von 1 g (3 mMol) 6-(2-Chlorphenyl)-1--methyl-4H-imidazo[1,5-a][1,4]benzodiazepin-3-carboxamid-5--oxid in 75 ml Essigsäureanhydrid wird 1 Stunde zum Rückfluss erhitzt. Ein Teil des Lösungsmittels (25 ml) wird mit einer Dean-Stark-Falle entfernt. Nach Eindampfen zur Trockene wird der Rückstand über 30 g Silicagel chromatographiert, wobei man Methylenchlorid/Aethylacetat 1:1 (V/V) als Eluierungsmittel verwendet. Die reinen Fraktionen des Produktes werden vereinigt und eingedampft. Kristallisation des Rückstandes aus Aether liefert N-Acetyl-4-acetyloxy-6--(2-chlorphenyl)-1-methyl-4H-imidazo[1,5-a][1,4]benzodiazepin-3-carboxamid als farblose Kristalle, Smp. 176-180°. NMR ($CDCl_3$) zeigt ein Gemisch von zwei Rotameren in einem ungefähren 1:1-Verhältnis (beschränktes Umklappen des 7-gliedrigen Ringes).

## Beispiel 72

6-(2-Chlorphenyl)-4-hydroxy-4H-imidazo[1,5-a][1,4]benzodiazepin-3-carboxamid

8,5 ml (17 mMol) 2N Natriumhydroxid werden zu einer Lösung von 0,75 g (1,7 mMol) 4-Acetyloxy-N-acetyl-6-(2--chlorphenyl)-4H-imidazo[1,5-a][1,4]benzodiazepin-3-carboxamid in 25 ml Methanol gegeben. Nach 1,5-stündigem Rühren bei Raumtemperatur unter Stickstoff wird die Lösung mit Kohlendioxid gepuffert und mit Wasser verdünnt. Die ausgefallenen Kristalle werden abfiltriert, mit Wasser gewaschen und trocken gesaugt. Umkristallisation aus Methylenchlorid/-Methanol liefert 6-(2-Chlorphenyl)-4-hydroxy-4H-imidazo-[1,5-a][1,4]benzodiazepin-3-carboxamid als farblose Kristalle, Smp. 305-308° (Zers.).

Beispiel 73

6-(2-Chlorphenyl)-4-hydroxy-1-methyl-4H-imidazo[1,5-a][1,4]-benzodiazepin-3-carboxamid

Eine Lösung von 3,4 g (7,5 mMol) N-Acetyl-4-acetyloxy--6-(2-chlorphenyl)-1-methyl-4H-imidazo[1,5-a][1,4]benzodiazepin-3-carboxamid in 40 ml Methanol wird mit 37,5 ml 2N Natriumhydroxidlösung versetzt. Nach 1-stündigem Rühren unter Stickstoff bei Raumtemperatur wird die Lösung mit Eisessig angesäuert und mit Wasser verdünnt. Das ausgefallene Produkt wird abfiltriert und aus Methylenchlorid/-Methanol umkristallisiert, wobei man 6-(2-Chlorphenyl)-4--hydroxy-1-methyl-4H-imidazo[1,5-a][1,4]benzodiazepin-3-carboxamid als farblose Kristalle erhält, Smp. 305-307°.

Beispiel 74

6-(2-Chlorphenyl)-4H-imidazo[1,5-a][1,4]benzodiazepin-3-carbonsäure-äthylester-5-oxid

(A) Ein Gemisch von 40,2 g (0,109 Mol) 6-(2-Chlorphenyl)--4H-imidazo[1,5-a][1,4]benzodiazepin-3-carbonsäure-äthylester, 35,6 g (0,165 Mol) 80%iger Chlorperbenzoesäure und 1 l Methylenchlorid wird 15 Minuten gerührt, bis der Festkörper gelöst ist und danach 18 Stunden bei Raumtemperatur stehengelassen. Nach Waschen mit 10%iger wässriger Natriumcarbonatlösung wird die Methylenchloridphase getrocknet und eingedampft. Kristallisation des Rückstandes aus 300 ml siedendem Methylacetat liefert 6-(2-Chlorphenyl)-4H-imidazo-[1,5-a][1,4]benzodiazepin-3-carbonsäure-äthylester-5-oxid, Smp. 242-245°.

(B) Diese Verbindung wird in 56%iger Ausbeute auch durch Umsetzen von 5-(2-Chlorphenyl)-1,3-dihydro-2H-1,4-benzodiazepin-2-on-4-oxid mit einer Base, gefolgt von Umsetzen mit Diäthylchlorphosphat und Aethylisocyanoacetat, erhalten.

## Beispiel 75

4-Acetyloxy-6-(2-chlorphenyl)-4H-imidazo[1,5-a][1,4]benzodiazepin-3-carbonsäure-äthylester

Ein Gemisch von 10 g (0,026 Mol) 6-(2-Chlorphenyl)-4H-imidazo[1,5-a][1,4]benzodiazepin-3-carbonsäure-äthylester-5-oxid und 140 ml Essigsäureanhydrid wird unter Rühren 1 Stunde zum Rückfluss erhitzt. Die Lösung wird dann auf ein kleines Volumen eingeengt, mit Aether verdünnt und in Eis gekühlt. Filtrieren und Waschen mit Aether liefert 4-Acetyloxy-6-(2-chlorphenyl)-4H-imidazo[1,5-a][1,4]benzodiazepin-3-carbonsäure-äthylester, Smp. 190-192°. Die Analysenprobe wird aus Aethylacetat/Hexan umkristallisiert und schmilzt bei 192-195°.

## Beispiel 76

6-(2-Chlorphenyl)-4-hydroxy-4H-imidazo[1,5-a][1,4]benzodiazepin-3-carboxamid

Ein Gemisch von 9,5 g (0,022 Mol) 4-Acetyloxy-6-(2-chlorphenyl)-4H-imidazo[1,5-a][1,4]benzodiazepin-3-carbonsäure-äthylester und 95 ml 20%igem methanolischem Ammoniak wird in einem geschlossenen Gefäss 23 Stunden bei Raumtemperatur gerührt. Filtrieren und Waschen mit Methanol liefert 6-(2-Chlorphenyl)-4-hydroxy-4H-imidazo[1,5-a][1,4]-benzodiazepin-3-carboxamid, Smp. 306-308°.

## Beispiel 77

6-(2-Chlorphenyl)-4-hydroxy-N-methyl-4H-imidazo[1,5-a][1,4]-benzodiazepin-3-carboxamid

Ein Gemisch von 3 g (0,007 Mol) 4-Acetyloxy-6-(2-chlorphenyl)-4H-imidazo[1,5-a][1,4]benzodiazepin-3-carbonsäure-äthylester und 45 ml 20%iger Methylaminlösung in Aethanol wird 18 Stunden in einem geschlossenen Gefäss bei Raumtemperatur gerührt. Das Produkt wird abfiltriert und mit Aethanol und Aether gewaschen, wobei man 6-(2-Chlorphenyl)-4-hydroxy-N-methyl-4H-imidazo[1,5-a][1,4]benzodiazepin-3-carboxamid erhält, Smp. 258-260°. Umkristallisation zu Analysenzwecken aus Tetrahydrofuran/Aethanol liefert farblose Kristalle, welche bei 260-262° schmelzen.

Beispiel 78

6-(2-Chlorphenyl)-N-äthyl-4-hydroxy-4H-imidazo[1,5-a][1,4]-benzodiazepin-3-carboxamid

(A) Ein Gemisch von 2 g 4-Acetyloxy-6-(2-chlorphenyl)-N--äthyl-4H-imidazo[1,5-a][1,4]benzodiazepin-3-carboxamid, 30 ml Methanol und 4 ml 3N Natriumhydroxidlösung wird 30 Minuten bei Raumtemperatur gerührt. Die Lösung wird dann zwischen Methylenchlorid und wässriger Natriumbicarbonatlösung verteilt. Die organische Phase wird getrocknet und eingedampft. Kristallisation des Rückstandes aus Methylenchlorid/Aethylacetat liefert 6-(2-Chlorphenyl)-N-äthyl-4--hydroxy-4H-imidazo[1,5-a][1,4]benzodiazepin-3-carboxamid als farblose Kristalle, Smp. 246-248°.

(B) Ein Gemisch von 0,43 g 4-Acetyloxy-6-(2-chlorphenyl)--4H-imidazo[1,5-a][1,4]benzodiazepin-3-carbonsäure-äthylester und 10 ml Aethanol enthaltend 20% (V/V) Aethylamin wird 24 Stunden bei Raumtemperatur gerührt. Das Lösungsmittel wird abgedampft, und der Rückstand aus Aethylenchlorid/-Aethylacetat umkristallisiert, wobei man 6-(2-Chlorphenyl)-N-äthyl-4-hydroxy-4H-imidazo[1,5-a][1,4]benzodiazepin-3-carboxamid als farbloses Produkt erhält, Smp. 246-248°.

Beispiel 79

4-Acetyloxy-6-(2-chlorphenyl)-N-äthyl-4H-imidazo[1,5-a][1,4]-benzodiazepin-3-carboxamid

Eine Lösung von 5,4 g 6-(2-Chlorphenyl)-N-äthyl-4H-imidazo[1,5-a][1,4]benzodiazepin-3-carboxamid-5-oxid in 70 ml Essigsäureanhydrid wird 18 Stunden auf 100° erhitzt. Das Reagens wird unter vermindertem Druck abgedampft, und der

Rückstand aus Aethylacetat/Aether/Hexan kristallisiert und aus Aethylacetat/Hexan umkristallisiert, wobei man 4-Acetyloxy-6-(2-chlorphenyl)-N-äthyl-4H-imidazo[1,5-a][1,4]benzodiazepin-3-carboxamid als farblose Kristalle erhält, Smp. 145-150°.

## Beispiel 80

6-(2-Chlorphenyl)-N-äthyl-4H-imidazo[1,5-a][1,4]benzodiazepin-3-carboxamid-5-oxid

Ein Gemisch von 6,5 g (0,017 Mol) 6-(2-Chlorphenyl)-N-äthyl-4H-imidazo[1,5-a][1,4]benzodiazepin-3-carboxamid, 4,75 g (0,22 Mol) 80%ige reine m-Chlorperbenzoesäure und 250 ml Methylenchlorid wird solange gerührt bis alles gelöst ist. Die Lösung wird danach 18 Stunden bei Raumtemperatur stehengelassen. Nach Waschen mit 10%iger wässriger Natriumcarbonatlösung wird die Methylenchloridlösung getrocknet und eingedampft. Der Rückstand wird unter Verwendung von einem Gemisch Aethanol/Methylenchlorid 1:19 an Silicagel chromatographiert. Die das Produkt enthaltenden Fraktionen werden vereinigt und eingedampft. Kristallisation aus Aethanol liefert 6-(2-Chlorphenyl)-N-äthyl-4H-imidazo[1,5-a][1,4]benzodiazepin-3-carboxamid-5-oxid, Smp. 175-180°.

## Beispiel 81

6-(2-Chlorphenyl)-1-(1,1-dimethyläthyl)-4H-imidazo[1,5-a]-[1,4]benzodiazepin-3-carbonsäure-(1,1-dimethyl)äthylester

Ein Gemisch von 7,95 g (0,02 Mol) 5-(2-Chlorphenyl)-α-(hydroxyimino)-3H-1,4-benzodiazepin-2-essigsäure-(1,1-dimethyl)äthylester, 8 g Raney-nickel, 150 ml Tetrahydrofuran, 75 ml Aethanol und 2 ml 20%igem methanolischem Ammoniak werden bei Atmosphärendruck 2,5 Stunden hydriert. Der Katalysator wird abfiltriert, und das Filtrat eingedampft. Der Rückstand wird in 200 ml Toluol gelöst, mit 5,16 g (0,06 Mol) Pivaldehyd versetzt und 1 Stunde gerührt. Nach

Zugabe von 8 g Mangandioxid wird eine weitere halbe Stunde gerührt. Danach wird das Mangandioxid abfiltriert, und das Filtrat eingedampft. Kristallisation aus Aether/Hexan liefert 6-(2-Chlorphenyl)-1-(1,1-dimethyläthyl)-4H-imidazo-[1,5-a][1,4]benzodiazepin-3-carbonsäure (1,1-dimethyl)-äthylester, Smp. 194-197°. Umkristallisation zu Analysenzwecken aus Aethylacetat/Hexan liefert ein Produkt, welches bei 195-197° schmilzt.

## Beispiel 82

6-(2-Chlorphenyl)-1-(1,1-dimethyläthyl)-4H-imidazo[1,5-a]-[1,4]benzodiazepin-3-carbonsäure

Eine Lösung von 1,1 g (2,5 mMol) 6-(2-Chlorphenyl)-1--(1,1-dimethyläthyl)-4H-imidazo[1,5-a][1,4]benzodiazepin-3--carbonsäure-(1,1-dimethyl)äthylester in 10 ml Trifluoressigsäure wird 2 Stunden bei Raumtemperatur stehengelassen und danach eingedampft. Das Oel wird mit Toluol azeotrop destilliert und danach aus siedendem Aethylacetat kristallisiert. Umkristallisation aus Methanol/Aethylacetat liefert 6-(2-Chlorphenyl)-1-(1,1-dimethyläthyl)-4H-imidazo-[1,5-a][1,4]benzodiazepin-3-carbonsäure.

## Beispiel 83

6-(2-Chlorphenyl)-1-(1,1-dimethyläthyl)-4H-imidazo[1,5-a]-[1,4]benzodiazepin-3-carboxamid

Eine Suspension von 0,56 g (1,4 mMol) 6-(2-Chlorphenyl)-1-(1,1-dimethyläthyl)-4H-imidazo[1,5-a][1,4]benzodiazepin-3-carbonsäure in 75 ml Methylenchlorid wird mit 0,38 g (1,8 mMol) Phosphorpentachlorid versetzt und dann 1 Stunde gerührt. Ammoniak wird solange in die Lösung eingeleitet, bis diese basisch reagiert. Nach Zugabe von 25 ml Wasser und 10 ml Aethanol und Rühren für 1 Stunde wird die Methylenchloridphase abgetrennt, getrocknet und eingedampft. Umkristallisation aus Methylenchlorid/Aethanol liefert 6-(2-Chlorphenyl)-1-(1,1-dimethyläthyl)-4H-imidazo-[1,5-a][1,4]benzodiazepin-3-carboxamid, Smp. 275-278°.

Beispiel 84

6-(2-Chlorphenyl)-1-propyl-4H-imidazo[1,5-a][1,4]benzodiazepin-3-carbonsäure-(1,1-dimethyl)äthylester

Ein Gemisch von 7,95 g (0,02 Mol) 5-(2-Chlorphenyl)-α-(hydroxyimino)-3H-1,4-benzodiazepin-2-essigsäure-(1,1-dimethyl)äthylester, 8 g Raney-nickel, 150 ml Tetrahydrofuran, 75 ml Aethanol und 2 ml 20%igem Ammoniak in Methanol wird 2,5 Stunden bei Raumtemperatur hydriert. Der Katalysator wird abfiltriert, und das Filtrat eingedampft. Der Rückstand wird in 200 ml Methylenchlorid gelöst, mit 4,32 g (0,06 Mol) Butyraldehyd versetzt, 1 Stunde in einem offenen Gefäss gerührt und danach eingedampft. Der Rückstand wird an Silicagel chromatographiert, wobei man ein 1:9-Gemisch Aethylacetat/Methylenchlorid als Eluierungsmittel verwendet. Die das gewünschte Produkt enthaltenden Fraktionen werden vereinigt und eingedampft. Kristallisation aus Aether liefert 6-(2-Chlorphenyl)-1-propyl-4H-imidazo-[1,5-a][1,4]benzodiazepin-3-carbonsäure-(1,1-dimethyl)äthylester, Smp. 176-179°. Für Analysenzwecke wird aus Aether umkristallisiert, wobei man ein Produkt erhält, welches bei 178-180° schmilzt.

Beispiel 85

6-(2-Chlorphenyl)-1-propyl-4H-imidazo[1,5-a][1,4]benzodiazepin-3-carbonsäure

Eine Lösung von 1,9 g (4,3 mMol) 6-(2-Chlorphenyl)-1-propyl-4H-imidazo[1,5-a][1,4]benzodiazepin-3-carbonsäure-(1,1-dimethyl)äthylester in 20 ml Trifluoressigsäure wird 2 Stunden bei Raumtemperatur stehengelassen und danach eingedampft und azeotrop mit Toluol destilliert. Kristallisation aus Methanol/Aethylacetat und Umkristallisation aus den gleichen Lösungsmitteln liefert 6-(2-Chlorphenyl)-1-

propyl-4H-imidazo[1,5-a][1,4]benzodiazepin-3-carbonsäure, Smp. 271-273°.

## Beispiel 86

6-(2-Chlorphenyl)-1-propyl-4H-imidazo[1,5-a][1,4]benzodiazepin-3-carboxamid

Eine Suspension von 1,1 g (2,8 mMol) 6-(2-Chlorphenyl)-1-propyl-4H-imidazo[1,5-a][1,4]benzodiazepin-3-carbonsäure in 125 ml Methylenchlorid wird mit 0,78 g (3,7 mMol) Phosphorpentachlorid versetzt und dann 1 Stunde gerührt. Ammoniak wird solange in die Lösung geleitet, bis diese basisch reagiert. Nach Zugabe von 50 ml Wasser und 25 ml Aethanol wird die Lösung 1 Stunde gerührt. Die Methylenchloridphase wird abgetrennt, getrocknet und eingedampft. Umkristallisation aus Methylenchlorid liefert 6-(2-Chlorphenyl)-1-propyl-4H-imidazo[1,5-a][1,4]benzodiazepin-3-carboxamid, Smp. 290-292°.

## Beispiel 87

5-(2-Chlorphenyl)-1,3-dihydro-2H-1,4-benzodiazepin-2-on 4-oxid

(A) 50 ml einer 1N Natriumhydroxidlösung wird zu einer Lösung von 5 g 2-Chlormethyl-4-(2-chlorphenyl)chinazolin-3-oxid in 250 ml Tetrahydrofuran gegeben. Nach 3-stündigem Rühren bei Raumtemperatur wird das Gemisch durch Zugabe von Eisessig angesäuert und auf ein kleines Volumen eingeengt. Dieses wird danach zwischen Methylenchlorid, enthaltend 10% Aethanol und wässriger Natriumbicarbonatlösung verteilt. Die organische Phase wird getrocknet und eingedampft, und der Rückstand aus Methylenchlorid/Aethanol umkristallisiert, wobei man 5-(2-Chlorphenyl)-1,3-dihydro-2H-1,4-benzodiazepin-2-on-4-oxid als farblose Kristalle erhält, Smp. 231-232°.

(B) Diese Verbindung wird auch in 88%iger Ausbeute erhalten, wenn man 5-(2-Chlorphenyl)-1,3-dihydro-2H-1,4-benzodiazepin-2-on mit m-Chlorperbenzoesäure in Methylenchlorid oxidiert.

## Beispiel 88

5-(2-Chlorphenyl)-1,3-dihydro-2H-1,4-benzodiazepin-2-ylidenpropandicarbonsäure-dimethylester

Eine Lösung von 27 g (0,1 Mol) 5-(2-Chlorphenyl)-1,3--dihydro-2H-1,4-benzodiazepin-2-on in 400 ml Tetrahydrofuran wird mit Eiswasser gekühlt. Nach Zugabe von 13 g (0,115 Mol) Kalium-t-butoxid wird das Gemisch 5 Minuten unter Stickstoff gerührt. Danach werden 18 ml (0,125 Mol) Diäthylchlorphosphat zugegeben und das Rühren für weitere 10 Minuten fortgeführt. Nach Zugabe von 26,4 g (0,2 Mol) Dimethylmalonat und 22,4 g (0,2 Mol) Kalium-t-butoxid wird das Gemisch ohne Kühlen 2 Stunden gerührt und danach durch Versetzen mit 20 ml Essigsäure angesäuert und zwischen Toluol und Natriumbicarbonatlösung verteilt. Die organische Phase wird abgetrennt, getrocknet und eingedampft. Kristallisation des Rückstandes aus Aether gibt gelbliche Kristalle, Smp. 133-138°. Zu Analysenzwecken wird das Produkt gereinigt, indem man es über Silicagel filtriert, wobei man 5% (V/V) Aethylacetat in Methylenchlorid als Eluierungsmittel verwendet. Kristallisation aus Aether liefert reines Material bestehend aus 5-(2-Chlorphenyl)-1,3-dihydro-2H--1,4-benzodiazepin-2-ylidenpropandicarbonsäure-dimethylester, Smp. 135-138°.

## Beispiel 89

5-(2-Chlorphenyl)-α-(hydroxyimino)-3H-1,4-benzodiazepin-2-essigsäure-methylester

Ein Gemisch von 9 g (23,4 mMol) 5-(2-Chlorphenyl)-1,3--dihydro-2H-1,4-benzodiazepin-2-ylidenpropandicarbonsäure-dimethylester, 6,6 g (0,117 Mol) Kaliumhydroxid und 300 ml

Methanol wird unter Stickstoff 3 Stunden zum Rückfluss erhitzt. Der Grossteil des Lösungsmittels wird unter vermindertem Druck entfernt, und der Rückstand zwischen Wasser und Methylenchlorid verteilt. Die organische Phase wird abgetrennt, getrocknet und eingedampft, wobei man öligen 5-(2-Chlorphenyl)-1,3-dihydro-2H-1,4-benzodiazepin-2-ylidenessigsäure-methylester erhält. Dieses Material wird in 50 ml Eisessig gelöst, und die Lösung mit 1,96 g (28 mMol) Natriumnitrit versetzt. Nach 1-stündigem Rühren bei Raumtemperatur wird das Gemisch mit Wasser verdünnt, und das ausgefallene Produkt abfiltriert, mit Wasser gewaschen und trocken gesaugt. Umkristallisation aus Methylenchlorid/-Aethanol liefert 5-(2-Chlorphenyl)-α-(hydroxyimino)-3H--1,4-benzodiazepin-2-essigsäure-methylester als gelbe Kristalle, Smp. 246-248°.

## Beispiel 90

6-(2-Chlorphenyl)-1-[(2-dimethylamino)äthyl]-4H-imidazo-[1,5-a][1,4]benzodiazepin-3-carbonsäure-methylester

Ein Gemisch von 8 g 5-(2-Chlorphenyl)-α-(hydroxyimino)-3H-1,4-benzodiazepin-2-essigsäure-methylester, 200 ml Tetrahydrofuran, 80 ml Methanol, 14 g Raney-nickel und 2 ml 20%igem methanolischem Ammoniak (V/V) wird bei Atmosphärendruck 2 Stunden hydriert. Der Katalysator wird abfiltriert, und das Filtrat eingedampft. Der Rückstand wird in 100 ml Methylenchlorid gelöst und mit 20 ml einer 20%igen (V/V) Lösung von Dimethylamin in Tetrahydrofuran und 4 ml Acrolein erhitzt. Nach 20-minütigem Rühren bei Raumtemperatur werden 14 g aktiviertes Mangandioxid zugegeben, und das Rühren 30 Minuten fortgeführt. Das Mangandioxid wird über Celit abfiltriert, und das Filtrat eingedampft. Kristallisation des Rückstands aus Aethylacetat/-Aether liefert 6-(2-Chlorphenyl)-1-[(2-dimethylamino)-äthyl]-4H-imidazo[1,5-a][1,4]benzodiazepin-3-carbonsäure--methylester, der aus Methanol/Aethylacetat/Aether umkristallisiert wird, wobei man farblose Kristalle erhält, Smp.

170-172°.

Beispiel 91

6-(2-Chlorphenyl)-1-[2-(dimethylamino)äthyl]-4H-imidazo-[1,5-a][1,4]benzodiazepin-3-carboxamid

Ein Gemisch von 3 g 6-(2-Chlorphenyl)-1-[2-(dimethylamino)äthyl]-4H-imidazo[1,5-a][1,4]benzodiazepin-3-carbonsäure-methylester, 3 g Ammoniumchlorid und 75 ml Methanol enthaltend 20% (V/V) Ammoniak werden 18 Stunden in einer Stahlbombe auf 100° erhitzt. Das Lösungsmittel wird teilweise abgedampft, und der Rückstand zwischen Methylenchlorid und wässrigem Ammoniak verteilt. Die organische Phase wird getrocknet und eingedampft. Der Rückstand wird aus Methanol/Aethylacetat kristallisiert, wobei man 6-(2-Chlorphenyl)-1-[2-(dimethylamino)äthyl]-4H-imidazo[1,5-a][1,4]-benzodiazepin-3-carboxamid erhält. Die Analysenprobe wird aus den gleichen Lösungsmitteln umkristallisiert, wobei man leicht gelbliche Kristalle erhält, Smp. 257-260°.

Beispiel 92

6-(2-Chlorphenyl)-1-(hydroxymethyl)-4H-imidazo[1,5-a][1,4] benzodiazepin-3-carbonsäure-(1,1-dimethyl)äthylester

Ein Gemisch von 1,9 g 6-(2-Chlorphenyl)-4H-imidazo-[1,5-a][1,4]benzodiazepin-1,3-dicarbonsäure-1-äthyl-3-(1,1-dimethyl)äthylester, 100 ml Aethanol und 1 g Natriumborhydrid wird 15 Minuten zum Rückfluss erhitzt. Das Lösungsmittel wird teilweise abgedampft, und der Rückstand zwischen Wasser und Methylenchlorid verteilt. Die organische Phase wird getrocknet und eingedampft. Kristallisation des Rückstands aus Methylenchlorid/Aethylacetat liefert 6-(2-Chlorphenyl)-1-(hydroxymethyl)-4H-imidazo[1,5-a][1,4]benzodiazepin-3-carbonsäure-(1,1-dimethyl)äthylester. Zu Analysenzwecken wird aus Methanol/Aethylacetat umkristallisiert, wobei man farblose Kristalle erhält, Smp. 253-254° (Zers.).

## Beispiel 93

1-(Chlormethyl)-6-(2-chlorphenyl)-4H-imidazo[1,5-a][1,4]-enzodiazepin-3-carbonsäure-(1,1-dimethyl)äthylester

6 g 6-(2-Chlorphenyl)-1-(hydroxymethyl)-4H-imidazo-[1,5-a][1,4]benzodiazepin-3-carbonsäure-(1,1-dimethyl)äthylester werden zu 30 ml Thionylchlorid gegeben. Nach 1-stündigem Rühren bei Raumtemperatur wird das Reagens abgedampft, und der Rückstand zwischen Methylenchlorid und 10%iger wässriger Natriumcarbonatlösung, welche Eis enthält, verteilt. Die organische Phase wird getrocknet und eingedampft. Der Rückstand wird aus Methylenchlorid/Aether kristallisiert, wobei man 1-(Chlormethyl)-6-(2-chlorphenyl)-4H-imidazo[1,5-a][1,4]benzodiazepin-3-carbonsäure--(1,1-dimethyl)äthylester erhält, Smp. 190-194° (Zers.). Die Analysenprobe wird gereinigt, indem man zunächst an der 30-fachen Menge Silicagel unter Verwendung von 10% (V/V) Aethylacetat in Methylenchlorid als Eluierungsmittel chromatographiert und danach aus Aethylacetat/Hexan kristallisiert, wobei man farblose Kristalle erhält, Smp. 194-195° (Zers.).

## Beispiel 94

6-(2-Chlorphenyl)-1-[(dimethylamino)methyl]-4H-imidazo-[1,5-a[]1,4]benzodiazepin-3-carbonsäure-(1,1-dimethyl)-äthylester

Ein Gemisch von 3 g 1-(Chlormethyl)-6-(2-chlorphenyl)--4H-imidazo[1,5-a][1,4]benzodiazepin-3-carbonsäure-(1,1-dimethyl)äthylester und 40 ml Tetrahydrofuran enthaltend 20% (V/V) Dimethylamin wird in einer Stahlbombe 3 Stunden auf 100° erhitzt. Das Lösungsmittel wird abgedampft, und der Rückstand zwischen Methylenchlorid und 10%iger wässriger Natriumcarbonatlösung verteilt. Die organische Phase wird getrocknet und eingedampft. Kristallisation des Rückstands aus Aethylacetat/Aether liefert 6-(2-Chlorphenyl)-1-[(dimethylamino)methyl]-4H-imidazo[1,5-a[]1,4]benzodiazepin-3-carbonsäure-(1,1-dimethyl)äthylester, Smp. 192-195°. Die Analysenprobe wird aus Aethylacetat/Hexan umkristallisiert, wobei man farblose Kristalle erhält, Smp. 198-200°.

## Beispiel 95

6-(2-Chlorphenyl)-1-[(dimethylamino)methyl]-4H-imidazo-[1,5-a][1,4]benzodiazepin-3-carbonsäure-methylester

Ein Gemisch von 2 g 6-(2-Chlorphenyl)-1-[(dimethylamino)methyl]-4H-imidazo[1,5-a][1,4]benzodiazepin-3-carbonsäure-(1,1-dimethyl)äthylester, 40 ml Methanol und 5 ml konz. Schwefelsäure wird 24 Stunden bei Raumtemperatur stehengelassen. Das Gemisch wird zwischen Methylenchlorid und mit Eis verdünntem Ammoniak verteilt. Die Methylenchloridphase wird abgetrennt, getrocknet und eingedampft. Der Rückstand wird mit 5% Aethanol in Methylenchlorid über Silicagel filtriert. Eindampfen und Kristallisation aus Aethylacetat/Aether liefert 6-(2-Chlorphenyl)-1-[(dimethylamino)methyl]-4H-imidazo[1,5-a][1,4]benzodiazepin-3-carbonsäure-methylester in zwei Fraktionen. Die Analysenprobe wird aus Aether umkristallsiert und schmilzt bei 184-185°. Man beobachtet auch eine tiefer schmelzende Modifikation, welche bei 166-169° schmilzt.

## Beispiel 96

6-(2-Chlorphenyl)-1-[(dimethylamino)methyl]-4H-imidazo-[1,5-a][1,4]benzodiazepin-3-carboxamid

Ein Gemisch von 2,5 g 6-(2-Chlorphenyl)-1-[(dimethylamino)methyl]-4H-imidazo[1,5-a][1,4]benzodiazepin-3-carbonsäure-methylester, 2,5 g Ammoniumchlorid und 40 ml Methanol enthaltend 20% (V/V) Ammoniak wird in einer Stahlbombe 18 Stunden auf 100° erhitzt. Das Lösungsmittel wird teilweise abgedampft, und der Rückstand zwischen Methylenchlorid und Wasser verteilt. Die organische Phase wird getrocknet und eingedampft. Der Rückstand wird an 50 g Silicagel unter Verwendung von 5% (V/V) Aethanol in Methylenchlorid

als Eluierungsmittel chromatographiert. Kristallisation aus Aethylacetat/Aether liefert 6-(2-Chlorphenyl)-1-[(dimethylamino)methyl]-4H-imidazo[1,5-a][1,4]benzodiazepin-3-carboxamid als farblose Kristalle, Smp. 228-230°.

Beispiel 97

6-(2-Chlorphenyl)-4H-imidazo[1,5-a][1,4]benzodiazepin-1,3-dicarbonsäure-1-äthyl-3-(1,1-dimethyl)äthylester

Ein Gemisch von 4 g (10 mMol) 5-(2-Chlorphenyl)-α--(hydroxyimino)-3H-1,4-benzodiazepin-2-essigsäure-(1,1-dimethyl)äthylester, 50 ml Tetrahydrofuran, 20 ml Aethanol, 1 ml Methanol enthaltend 20% (V/V) Ammoniak und 5 g Raney--nickel werden bei Atmosphärendruck solange hydriert, bis die Wasserstoffaufnahme aufgehört hat (2 Stunden). Der Katalysator wird abfiltriert, und das Filtrat eingedampft. Der Rückstand wird in 100 ml Methylenchlorid gelöst, mit 2,5 g Aethylglyoxalat versetzt und in einem offenen Gefäss 18 Stunden bei Raumtemperatur gerührt. Das Gemisch wird über Silicagel filtriert, wobei man 5% (V/V) Aethylacetat in Methylenchlorid als Eluierungsmittel verwendet. Das Filtrat wird eingedampft, und der Rückstand aus Aether kristallisiert, wobei man 6-(2-Chlorphenyl)-4H-imidazo[1,5-a]-[1,4]benzodiazepin-1,3-dicarbonsäure-1-äthyl-3-(1,1-dimethyl)äthylester erhält. Die Analysenprobe wird aus Aethylacetat/Hexan umkristallisiert, wobei man farblose Kristalle erhält, Smp. 185-187°.

## Beispiel A

Mikronisiertes 6-(2-Chlorphenyl)-4-hydroxy-4H-imidazo-[1,5-a][1,4]benzodiazepin-3-carboxamid kann als Wirkstoff zur Herstellung von Arzneiformen folgender Zusammensetzung verwendet werden:

a) Tabletten (direkte Verpressung)

| | Bestandteile | mg/Tablette | | | | |
|---|---|---|---|---|---|---|
| 1. | Wirkstoff | 0.1 | 0.5 | 5,0 | 10.0 | 25.0 |
| 2. | Milchzucker | 85.4 | 85.5 | 81.0 | 103.0 | 112.5 |
| 3. | Avicel | 30.0 | 30.0 | 30.0 | 45.0 | 60.0 |
| 4. | Modifizierte Stärke | 8 | 7.5 | 7.5 | 10.0 | 15.0 |
| 5. | Magnesiumstearat | 1.5 | 1.5 | 1.5 | 2.0 | 2.5 |
| | Total | 125 | 125 | 125 | 170 | 215 |

b) Tabletten (direkte Verpressung)

| | Bestandteile | mg/Tablette | | |
|---|---|---|---|---|
| 1. | Wirkstoff | 10.0 | 20.0 | 40.0 |
| 2. | Milchzucker | 182.0 | 172.0 | 216.0 |
| 3. | Mikrokristalline Cellulose | 60.0 | 60.0 | 80.0 |
| 4. | Modifizierte Stärke | 15.0 | 15.0 | 20.0 |
| 5. | Maisstärke | 30.0 | 30.0 | 40.0 |
| 6. | Magnesiumstearat | 3.0 | 3.0 | 4.0 |
| | Total | 300 | 300 | 400 |

c) Kapseln:

| | Bestandteile | mg/Kapsel | | | | |
|---|---|---|---|---|---|---|
| 1. | Wirkstoff | 0.1 | 0.5 | 5.0 | 10.0 | 25.0 |
| 2. | Milchzucker | 183.9 | 183.5 | 179.0 | 218.0 | 257.0 |
| 3. | Stärke | 30.0 | 30.0 | 30.0 | 50.0 | 70.0 |
| 4. | Talk | 5.0 | 5.0 | 5.0 | 10.0 | 15.0 |
| 5. | Magnesiumstearat | 1.0 | 1.0 | 1.0 | 2.0 | 3.0 |
| | Total | 220 | 220 | 220 | 290 | 370 |

d) Kapseln:

| | Bestandteile | mg/ Kapsel | | |
|---|---|---|---|---|
| 1. | Wirkstoff | 10.0 | 20.0 | 40.0 |
| 2. | Milchzucker | 215.0 | 205.0 | 260.0 |
| 3. | Maisstärke | 60.0 | 60.0 | 80.0 |
| 4. | Magnesiumstearat | 3.0 | 3.0 | 4.0 |
| 5. | Talk | 12.0 | 12.0 | 16.0 |
| | Total | 300 | 300 | 400 |

## Beispiel B

Mikronisiertes 6-(2-Chlorphenyl)-4H-imidazo[1,5-a]-[1,4]benzodiazepin-3-carboxamid kann als Wirkstoff zur Herstellung von Arzneiformen der folgenden Zusammensetzung verwendet werden:

a) Tabletten (direkte Verpressung)

| | Bestandteile | mg/Tablette | | | | |
|---|---|---|---|---|---|---|
| 1. | Wirkstoff | 0.1 | 0.5 | 5.0 | 10.0 | 25.0 |
| 2. | Milchzucker | 85.4 | 85.5 | 81.0 | 103.0 | 112.5 |
| 3. | Avicel | 30.0 | 30.0 | 30.0 | 45.0 | 60.0 |
| 4. | Modifizierte Stärke | 8 | 7.5 | 7.5 | 10.0 | 15.0 |
| 5. | Magnesiumstearat | 1.5 | 1.5 | 1.5 | 2.0 | 2.5 |
| | Total | 125 | 125 | 125 | 170 | 215 |

b) Tabletten (direkte Verpressung)

| | Bestandteile | mg/Tablette | | |
|---|---|---|---|---|
| 1. | Wirkstoff | 10.0 | 20.0 | 40.0 |
| 2. | Milchzucker | 182.0 | 172.0 | 216.0 |
| 3. | Mikrokristalline Cellulose | 60.0 | 60.0 | 80.0 |
| 4. | Modifizierte Stärke | 15.0 | 15.0 | 20.0 |
| 5. | Maisstärke | 30.0 | 30.0 | 40.0 |
| 6. | Magnesiumstearat | 3.0 | 3.0 | 4.0 |
| | Total | 300 | 300 | 400 |

c) Kapseln:

| | Bestandteile | mg/Kapsel | | | | |
|---|---|---|---|---|---|---|
| 1. | Wirkstoff | 0.1 | 0.5 | 5.0 | 10.0 | 25.0 |
| 2. | Milchzucker | 183.9 | 183.5 | 179.0 | 218.0 | 257.0 |
| 3. | Stärke | 30.0 | 30.0 | 30.0 | 50.0 | 70.0 |
| 4. | Talk | 5.0 | 5.0 | 5.0 | 10.0 | 15.0 |
| 5. | Magnesiumstearat | 1.0 | 1.0 | 1.0 | 2.0 | 3.0 |
| | Total | 220 | 220 | 220 | 290 | 370 |

d) Kapseln:

| | Bestandteile | mg/Kapsel | | |
|---|---|---|---|---|
| 1. | Wirkstoff | 10.0 | 20.0 | 40.0 |
| 2. | Milchzucker | 215.0 | 205.0 | 260.0 |
| 3. | Maisstärke | 60.0 | 60.0 | 80.0 |
| 4. | Magnesiumstearat | 3.0 | 3.0 | 4.0 |
| 5. | Talk | 12.0 | 12.0 | 16.0 |
| | Total | 300 | 300 | 400 |

## Beispiel C

Mikronisiertes 9-Chlor-6-(2-chlorphenyl)-4H-imidazo-[1,5-a][1,4]benzodiazepin-3-carboxamid kann als Wirkstoff zur Herstellung von Arzneiformen der folgenden Zusammensetzung verwendet werden:

a) Tabletten (direkte Verpressung)

| | Bestandteile | mg/Tablette | | | | |
|---|---|---|---|---|---|---|
| 1. | Wirkstoff | 0.1 | 0.5 | 5.0 | 10.0 | 25.0 |
| 2. | Milchzucker | 85.4 | 85.5 | 81.0 | 103.0 | 112.5 |
| 3. | Avicel | 30.0 | 30.0 | 30.0 | 45.0 | 60.0 |
| 4. | Modifizierte Stärke | 8 | 7.5 | 7.5 | 10.0 | 15.0 |
| 5. | Magnesiumstearat | 1.5 | 1.5 | 1.5 | 2.0 | 2.5 |
| | Total | 125 | 125 | 125 | 170 | 215 |

b) Tabletten (direkte Verpressung)

| | Bestandteile | mg/Tablette | | |
|---|---|---|---|---|
| 1. | Wirkstoff | 10.0 | 20.0 | 40.0 |
| 2. | Milchzucker | 182.0 | 172.0 | 216.0 |
| 3. | Mikrokristalline Cellulose | 60.0 | 60.0 | 80.0 |
| 4. | Modifizierte Stärke | 15.0 | 15.0 | 20.0 |
| 5. | Maisstärke | 30.0 | 30.0 | 40.0 |
| 6. | Magnesiumstearat | 3.0 | 3.0 | 4.0 |
| | Total | 300 | 300 | 400 |

c) Kapseln:

| | Bestandteile | mg/Kapsel | | | | |
|---|---|---|---|---|---|---|
| 1. | Wirkstoff | 0.1 | 0.5 | 5.0 | 10.0 | 25.0 |
| 2. | Milchzucker | 183.9 | 183.5 | 179.0 | 218.0 | 257.0 |
| 3. | Stärke | 30.0 | 30.0 | 30.0 | 50.0 | 70.0 |
| 4. | Talk | 5.0 | 5.0 | 5.0 | 10.0 | 15.0 |
| 5. | Magnesiumstearat | 1.0 | 1.0 | 1.0 | 2.0 | 3.0 |
| | Total | 220 | 220 | 220 | 290 | 370 |

d) Kapseln:

| | Bestandteile | mg/Kapsel | | |
|---|---|---|---|---|
| 1. | Wirkstoff | 10.0 | 20.0 | 40.0 |
| 2. | Milchzucker | 215.0 | 205.0 | 260.0 |
| 3. | Maisstärke | 60.0 | 60.0 | 80.0 |
| 4. | Magnesiumstearat | 3.0 | 3.0 | 4.0 |
| 5. | Talk | 12.0 | 12.0 | 16.0 |
| | Total | 300 | 300 | 400 |

## Patentansprüche

1. Imidazobenzodiazepinderivate der allgemeinen Formel

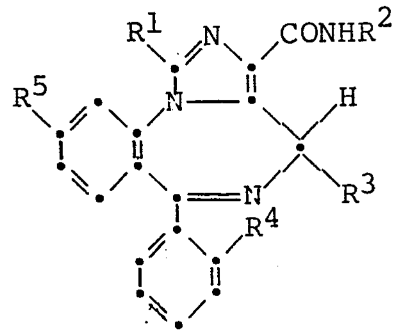

I

worin $R^1$ Wasserstoff, Di-nieder Alkylamino-nieder Alkyl oder nieder Alkyl, $R^2$ Wasserstoff nieder Alkanoyl oder nieder Alkyl, $R^3$ Wasserstoff, nieder Alkanoyloxy oder Hydroxy, $R^4$ Chlor, Brom, Fluor oder Trifluormethyl und $R^5$ Wasserstoff, Fluor oder Chlor bedeuten, mit den Massgaben, dass a) $R^3$ nicht Hydroxy bedeuten kann, falls $R^2$ nieder Alkanoyl bedeutet und b) $R^3$ Wasserstoff bedeutet, falls $R^1$ Di-nieder Alkylamino-nieder Alkyl bedeutet,
und pharmazeutisch verwendbare Salze davon.

2. Verbindungen gemäss Anspruch 1, worin $R^1$ Wasserstoff bedeutet.

3. Verbindungen gemäss Anspruch 1 oder 2, worin $R^2$ Wasserstoff oder Aethyl bedeutet.

4. Verbindungen gemäss einem der Ansprüche 1-3, worin $R^4$ Chlor oder Fluor bedeutet.

5. Verbindungen gemäss einem der Ansprüche 1-4, worin $R^5$ Wasserstoff bedeutet.

6. 9-Chlor-6-(2-chlorphenyl)-4H-imidazo[1,5-a][1,4]-benzodiazepin-3-carboxamid.

7. 6-(2-Chlorphenyl)-4H-imidazo[1,5-a][1,4]benzodiazepin-3-carboxamid.

8. 6-(2-Chlorphenyl)-1-methyl-4H-imidazo[1,5-a][1,4]-benzodiazepin-3-carboxamid.

9. 6-(2-Chlorphenyl)-4-hydroxy-4H-imidazo[1,5-a][1,4]-benzodiazepin-3-carboxamid.

10. N-Acetyl-6-(2-chlorphenyl)-4H-imidazo[1,5-a][1,4]-benzodiazepin-3-carboxamid.

11. Eine Verbindung gemäss einem der Ansprüche 1-10 oder ein pharmazeutisch verwendbares Salz davon zur Anwendung als therapeutischer Wirkstoff.

12. Eine Verbindung gemäss einem der Ansprüche 1-10 oder ein pharmazeutisch verwendbares Salz davon zur Anwendung als Anxiolytikum.

13. Verfahren zur Herstellung von Verbindungen der Formel I in Anspruch 1 und pharmazeutisch verwendbaren Salzen davon, dadurch gekennzeichnet, dass man

a) eine Verbindung der allgemeinen Formel

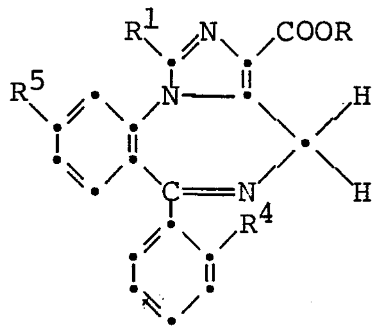

XII

worin R niederes Alkyl bedeutet und $R^1$, $R^4$ und $R^5$ obige Bedeutung besitzen,

einer Aminolyse mit einer Aminoverbindung der Formel $H_2NR^{21}$, worin $R^{21}$ Wasserstoff oder niederes Alkyl bedeutet, unterwirft, oder

b) eine Verbindung der allgemeinen Formel

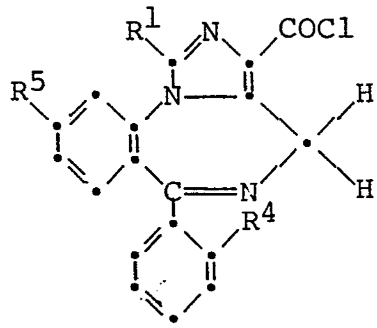

XVI

worin $R^1$, $R^4$ und $R^5$ die obige Bedeutung besitzen, einer Aminolyse mit einer Aminoverbindung der Formel $H_2NR^{21}$, worin $R^{21}$ obige Bedeutung besitzt, unterwirft, oder

c) eine Verbindung der allgemeinen Formel

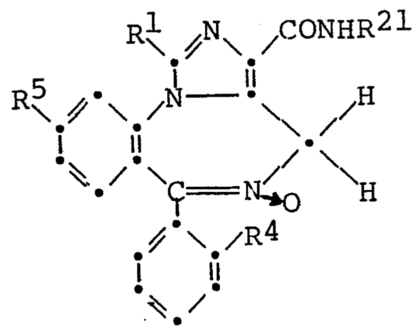

XVII

worin $R^1$, $R^{21}$, $R^4$ und $R^5$ die obige Bedeutung besitzen,

mit einem $C_1$-$C_4$-Carbonsäureanhydrid umsetzt, oder

d) eine Verbindung der allgemeinen Formel

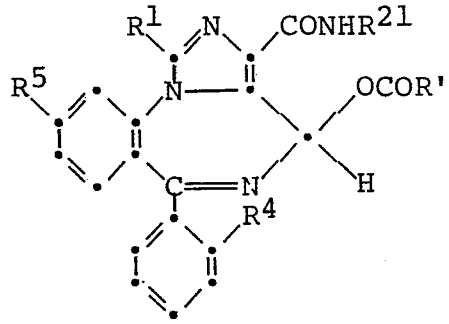

Ib'

worin R' $C_1$-$C_4$-Alkyl bedeutet und $R^1$, $R^{21}$, $R^4$ und $R^5$ die obige Bedeutung besitzen,

mit einem Alkalimetallhydroxyd umsetzt, oder

e) eine Verbindung der allgemeinen Formel

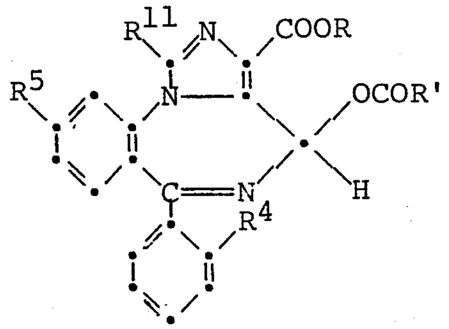

XVIII

worin $R^{11}$ Wasserstoff oder nieder Alkyl bedeutet und R, R', $R^4$ und $R^5$ obige Bedeutung besitzen,

einer Aminolyse mit einer Aminoverbindung der Formel $H_2NR^{21}$, worin $R^{21}$ die obige Bedeutung besitzt, unterwirft, oder

f) eine Verbindung der allgemeinen Formel

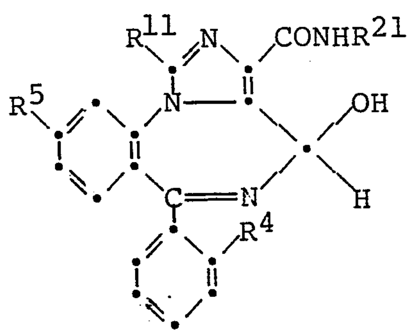

Ib''

worin $R^{11}$, $R^{21}$, $R^4$ und $R^5$ obige Bedeutung besitzen,

mit einem $C_1$-$C_4$-Carbonsäurechlorid oder -anhydrid umsetzt, oder

g) eine Verbindung der allgemeinen Formel

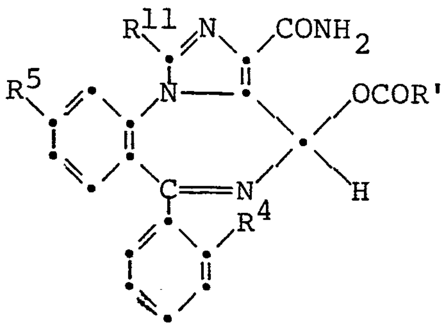

Ic'

worin R', $R^{11}$, $R^4$ und $R^5$ die obige Bedeutung besitzen,

mit einem $C_1$-$C_4$-Carbonsäurechlorid oder -anhydrid umsetzt, und

h) erwünschtenfalls eine erhaltene Verbindung der Formel I in ein pharmazeutisch verwendbares Salz überführt.

14. Arzneimittel enthaltend eine Verbindung gemäss einem der Ansprüche 1-10 oder ein pharmazeutisch verwendbares Salz davon und ein therapeutisch inertes Excipiens.

15. Anxiolytikum enthaltend eine Verbindung gemäss einem der Ansprüche 1-10 oder ein pharmazeutisch verwendbares Salz davon und ein therapeutisch inertes Excipiens.

***

Patentansprüche für Oesterreich

1. Verfahren zur Herstellung von Imidazobenzodiazepinderivaten der allgemeinen Formel

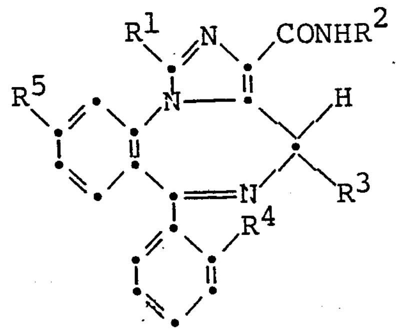

I

worin $R^1$ Wasserstoff, Di-nieder Alkylamino-nieder Alkyl oder nieder Alkyl, $R^2$ Wasserstoff nieder Alkanoyl oder nieder Alkyl, $R^3$ Wasserstoff, nieder Alkanoyloxy oder Hydroxy, $R^4$ Chlor, Brom, Fluor oder Trifluormethyl und $R^5$ Wasserstoff, Fluor oder Chlor bedeuten, mit den Massgaben, dass a) $R^3$ nicht Hydroxy bedeuten kann, falls $R^2$ nieder Alkanoyl bedeutet und b) $R^3$ Wasserstoff bedeutet, falls $R^1$ Di-nieder Alkylamino-nieder Alkyl bedeutet,

und pharmazeutisch verwendbaren Salzen davon, dadurch gekennzeichnet, dass man

a) eine Verbindung der allgemeinen Formel

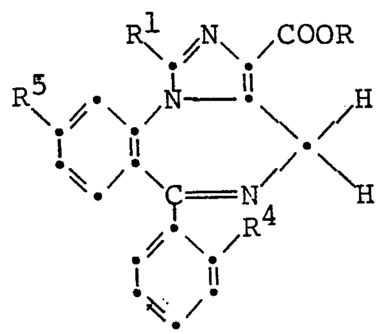

XII

worin R niederes Alkyl bedeutet und $R^1$, $R^4$ und $R^5$ obige Bedeutung besitzen,

einer Aminolyse mit einer Aminoverbindung der Formel $H_2NR^{21}$, worin $R^{21}$ Wasserstoff oder niederes Alkyl bedeutet, unterwirft, oder

b) eine Verbindung der allgemeinen Formel

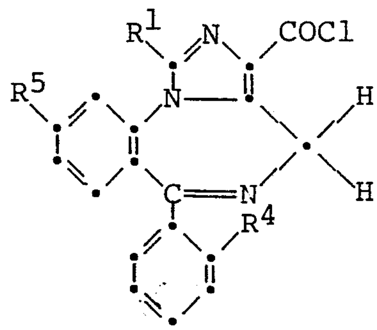

XVI

worin $R^1$, $R^4$ und $R^5$ die obige Bedeutung besitzen, einer Aminolyse mit einer Aminoverbindung der Formel $H_2NR^{21}$, worin $R^{21}$ obige Bedeutung besitzt, unterwirft, oder

c) eine Verbindung der allgemeinen Formel

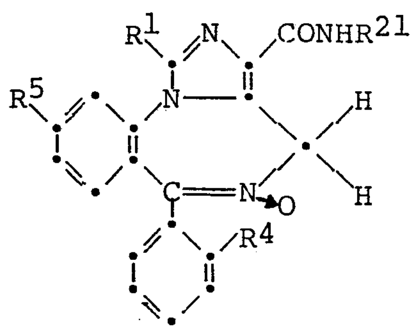

XVII

worin $R^1$, $R^{21}$, $R^4$ und $R^5$ die obige Bedeutung besitzen,

mit einem $C_1$-$C_4$-Carbonsäureanhydrid umsetzt, oder

d) eine Verbindung der allgemeinen Formel

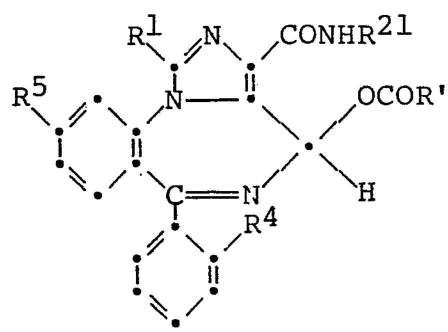

Ib'

worin R' $C_1$-$C_4$-Alkyl bedeutet und $R^1$, $R^{21}$, $R^4$ und $R^5$ die obige Bedeutung besitzen,
mit einem Alkalimetallhydroxyd umsetzt, oder

e) eine Verbindung der allgemeinen Formel

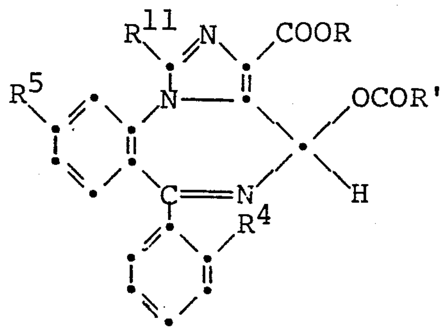

XVIII

worin $R^{11}$ Wasserstoff oder nieder Alkyl bedeutet und R, R', $R^4$ und $R^5$ obige Bedeutung besitzen,
einer Aminolyse mit einer Aminoverbindung der Formel $H_2NR^{21}$, worin $R^{21}$ die obige Bedeutung besitzt, unterwirft, oder

f) eine Verbindung der allgemeinen Formel

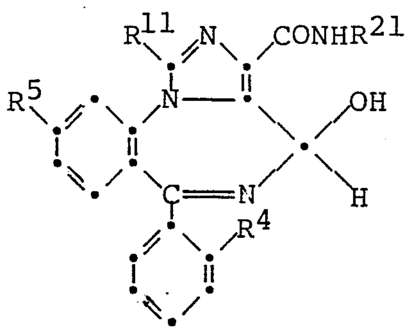

Ib''

worin $R^{11}$, $R^{21}$, $R^4$ und $R^5$ obige Bedeutung besitzen,
mit einem $C_1$-$C_4$-Carbonsäurechlorid oder -anhydrid umsetzt, oder

g) eine Verbindung der allgemeinen Formel

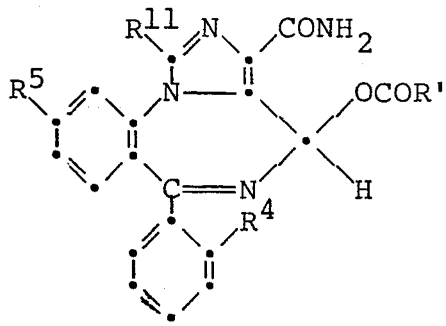

Ic'

worin R', $R^{11}$, $R^4$ und $R^5$ die obige Bedeutung besitzen,

mit einem $C_1$-$C_4$-Carbonsäurechlorid oder -anhydrid umsetzt, und

h) erwünschtenfalls eine erhaltene Verbindung der Formel I in ein pharmazeutisch verwendbares Salz überführt.

2. Verfahren gemäss Anspruch 1, dadurch gekennzeichnet, dass $R^1$ Wasserstoff bedeutet.

3. Verfahren gemäss Anspruch 1 oder 2, dadurch gekennzeichnet, dass $R^2$ Wasserstoff oder Aethyl bedeutet.

4. Verfahren gemäss einem der Ansprüche 1-3, dadurch gekennzeichnet, dass $R^4$ Chlor oder Fluor bedeutet.

5. Verfahren gemäss einem der Ansprüche 1-4, dadurch gekennzeichnet, dass $R^5$ Wasserstoff bedeutet.

6. Verfahren gemäss Anspruch 1, dadurch gekennzeichnet, dass man 9-Chlor-6-(2-chlorphenyl)-4H-imidazo[1,5-a][1,4]-benzodiazepin-3-carboxamid herstellt.

7. Verfahren gemäss Anspruch 1, dadurch gekennzeichnet, dass man 6-(2-Chlorphenyl)-4H-imidazo[1,5-a][1,4]benzodiazepin-3-carboxamid herstellt.

8. Verfahren gemäss Anspruch 1, dadurch gekennzeichnet, dass man 6-(2-Chlorphenyl)-1-methyl-4H-imidazo[1,5-a][1,4]-benzodiazepin-3-carboxamid herstellt.

9. Verfahren gemäss Anspruch 1, dadurch gekennzeichnet, dass man 6-(2-Chlorphenyl)-4-hydroxy-4H-imidazo[1,5-a]-[1,4]benzodiazepin-3-carboxamid herstellt.

10. Verfahren gemäss Anspruch 1, dadurch gekennzeichnet, dass man N-Acetyl-6-(2-chlorphenyl)-4H-imidazo[1,5-a]-[1,4]benzodiazepin-3-carboxamid herstellt.